# EUROPEAN PATENT APPLICATION

(11) **EP 2 243 828 A1**
(43) Date of publication of application: **27.10.2010**
(21) Application number: 09158709.7
(22) Date of filing: 24.04.2009
(51) Int. Cl.: C12N 9/12, A61K 38/45

(54) **Use of mixed lineage like kinase polypeptides (MLKL polypeptides) in cancer therapy**

(71) Applicant: DKFZ Deutsches Krebsforschungszentrum, 69120 Heidelberg (DE)
(72) Inventor: Mollenhauer, Jan, 5540 Ullerslev (DK); Hudler, Melanie, 69221 Dossenheim (DE); Blaich, Stephanie, 69121 Heidelberg (DE); Wittig, Rainer, 89077 Ulm (DE)
(74) Representative: Stößel, Matthias

(57) **Abstract**

The present invention pertains to the field of therapeutic and diagnostic compositions. Specifically, it relates to a polynucleotide comprising a nucleic acid sequence encoding a MLKL polypeptide or variants thereof for use as medicament. The present invention relates further to a nucleic acid which specifically binds to a polynucleotide encoding a MLKL polypeptide for use as a medicament, whereby the nucleic acid upon specific binding prevents the generation of a biologically active polypeptide encoded by the said polynucleotide. Additionally, the present invention relates to an antibody or an RNA-aptamer which specifically recognizes a polypeptide encoded by a polynucleotide as specified above for use as a medicament. The present invention also relates to the use of a polynucleotide of the present invention, a nucleic acid of the present invention, or an antibody of the present invention for the manufacture of a medicament for the treatment of cancer. The present invention relates to methods for diagnosing cancer in a subject, methods for assessing whether a subject suffering from cancer will benefit from a MLKL based anti-cancer treatment, methods for identifying MLKL modulators, and a method for the manufacture of medicament comprising the steps of the aforementioned methods and a further step of formulating the modulator as a medicament.

## Description

The present invention pertains to the field of therapeutic and diagnostic compositions. Specifically, it relates to a polynucleotide comprising a nucleic acid sequence encoding a MLKL polypeptide or variants thereof for use as medicament. The present invention relates further to a nucleic acid which specifically binds to a polynucleotide encoding a MLKL polypeptide for use as a medicament, whereby the nucleic acid upon specific binding prevents the generation of a biologically active polypeptide encoded by the said polynucleotide. Additionally, the present invention relates to an antibody or an RNA-aptamer which specifically recognizes a polypeptide encoded by a polynucleotide as specified above for use as a medicament. The present invention also relates to the use of a polynucleotide of the present invention, a nucleic acid of the present invention, or an antibody of the present invention for the manufacture of a medicament for the treatment of cancer. The present invention relates to methods for diagnosing cancer in a subject, methods for assessing whether a subject suffering from cancer will benefit from a MLKL based anti-cancer treatment, methods for identifying MLKL modulators, and a method for the manufacture of medicament comprising the steps of the aforementioned methods and a further step of formulating the modulator as a medicament.

Despite of steady improvements in cancer therapy, only a subset of the tumor patients can be treated successfully. Cancer remains one of the major causes of mortality. Kinases are critical modulators of signaling processes and many kinases have been linked to tumorigenesis, see Blume-Jensen, 2001, Nature 411, 355; Hunter, 2000, Cell 100, 113. However, only a few of the approximately 500 kinases within the human genome play a role in cancer as oncogenes. In addition, most of these 500 genes are only assigned as kinases based on homology data only and without knowledge about their functions or their relevance for cancer or other diseases, see Manning, 2002 Science 298, 1912-1934 Cancer is caused by alterations in oncogenes, tumor-suppressor genes, and microRNA genes. These alterations are usually somatic events, although germ-line mutations can predispose a person to heritable or familial cancer. A single genetic change is rarely sufficient for the development of a malignant tumor. Rather, the current evidence points pivotally to a multistep process of sequential alterations in several oncogenes, tumor-suppressor genes, or microRNA genes in cancer cells. Oncogenes encode proteins that control cell proliferation, apoptosis, or both. They can be activated by amplification, by structural alterations resulting from mutation or gene fusion by juxtaposition to enhancer elements. Translocations and mutations can occur as initiating events or during tumor progression, whereas amplification usually occurs during progression. The products of oncogenes can be classified into six broad groups: transcription factors, chromatin remodelers, growth factors, growth factor receptors, signal transducers, and apoptosis regulators, see Croce 2008, N Engl J Med 358, 502-511. Many oncogenes encode members of signal-transduction pathways. They fall into two main groups: non-receptor protein kinases and guanosine-triphosphate-binding proteins. The non-receptor protein kinases are of two types: tyrosine kinases (e.g., ABL, LCK, and SRC) and serine and threonine kinases (e.g., AKT, RAF1, MOS, and PIM1). Proteins involved in signal transduction become oncogenic if they bear activating mutations. An important example is PI3K and some of its downstream targets, such as AKT and SGK, which are critical to tyrosine kinase signaling and can be mutated in cancer cells, see Croce 2008, N Engl J Med 358, 502-511.

The identification of oncogenes involved in the initiation and progression of tumors can generate targets for the development of new anticancer drugs. The advantage of targeted therapy is the dependency of cancer cells on the oncogene product for growth and survival. Thus, cancer cells are more sensitive to the treatment than are normal cells. Furthermore, it is well documented that inhibition of oncogenes exerts positive therapeutic effects, see Daucey and Sausville, 2003, Nat. Rev Drug Discov 4, 296-313 ; Harari, 2004, Endocr Relat Cancer, 4, 689-708; Press and Lenz, 2007, Drugs, 14, 2045-75. Moreover, they can be used as tools for diagnosing a tumor positive for the respective oncogene and for predicting whether they can be classified into a group of potentially treatment responsive tumors.

Currently, there is a strong need for new anticancer treatments. Thus, identifying oncogenes for the use as new drug targets for cancer treatment is of particular importance but still, rather difficult to achieve. Means and methods for the development of new drugs for cancer treatment are highly desirable, but strongly depend on the availability of oncogenes that represent suitable drug targets.

Thus, the technical problem underlying the present invention may be seen as the provision of means and methods for complying with the aforementioned needs. The technical problem is solved by the embodiments characterized in the claims and herein below.

The present invention relates to a polynucleotide comprising a nucleic acid sequence selected from the group consisting of:
a) a nucleic acid sequence as shown in SEQ ID NOs: 1, 3, 5, 7, 9, 11, or 13,
b) a nucleic acid sequence encoding a polypeptide having an amino acid sequence as shown in SEQ ID NOs: 2, 4, 6, 8 10, 12, or 14,
c) a nucleic acid sequence which is at least 60% identical to the nucleic acid sequence of (a), and
d) a nucleic acid sequence encoding for a polypeptide having an amino acid sequence which is at least 60% identical to the amino acid sequence of (b)
for use as a medicament.

The term "polynucleotide" as used in accordance with the present invention relates to a polynucleotide comprising a nucleic acid sequence which encodes a mixed lineage kinase domain-like (MLKL) polypeptide having protein kinase activity. The polynucleotide, preferably, comprises or essentially consists of a nucleic acid sequence as shown in SEQ ID NO: 1, 3, 5, 7, 9, 11, or 13, or a nucleic acid sequence encoding a polypeptide having an amino acid sequence as shown in SEQ ID NO: 2, 4, 6, 8, 10, 12, or 14. Moreover, variants of said polynucleotide are encompassed. Said variants may represent orthologs, paralogs or other homologs of said polynucleotide. The polynucleotide variants, preferably, comprise a nucleic acid sequence **characterized in that** the sequence can be derived from the aforementioned specific nucleic acid sequences shown in SEQ ID NOs: 1, 3, 5, 7, 9, 11 or 13 by at least one nucleotide substitution, addition and/or deletion whereby the variant nucleic acid sequence shall still encode a polypeptide having the activity as specified above. Variants also encompass polynucleotides comprising a nucleic acid sequence which is capable of hybridizing to the aforementioned specific nucleic acid sequence, preferably, under stringent hybridization conditions. These stringent conditions are known to the skilled worker and can be found in Current Protocols in Molecular Biology, John Wiley & Sons, N. Y. (1989), 6.3.1-6.3.6. A preferred example for stringent hybridization conditions are hybridization conditions in 6 x sodium chloride/sodium citrate (= SSC) at approximately 45°C, followed by one or more wash steps in 0.2 x SSC, 0.1% SDS at 50 to 65°C. The skilled worker knows that these hybridization conditions differ depending on the type of nucleic acid and, for example when organic solvents are present, with regard to the temperature and concentration of the buffer. For example, under "standard hybridization conditions" the temperature differs depending on the type of nucleic acid between 42°C and 58°C in aqueous buffer with a concentration of 0.1 to 5 x SSC (pH 7.2). If organic solvent is present in the abovementioned buffer, for example 50% formamide, the temperature under standard conditions is approximately 42°C. The hybridization conditions for DNA: DNA hybrids are preferably for example 0.1 x SSC and 20°C to 45°C, preferably between 30°C and 45°C. The hybridization conditions for DNA: RNA hybrids are preferably, for example, 0.1 x SSC and 30°C to 55°C, preferably between 45°C and 55°C. The abovementioned hybridization temperatures are determined for example for a nucleic acid with approximately 100 bp (= base pairs) in length and a G + C content of 50% in the absence of formamide. The skilled worker knows how to determine the hybridization conditions required by referring to textbooks such as the textbook mentioned above, or the following textbooks: Sambrook et al., "Molecular Cloning", Cold Spring Harbor Laboratory, 1989; Hames and Higgins (Ed.) 1985, "Nucleic Acids Hybridization: A Practical Approach", IRL Press at Oxford University Press, Oxford; Brown (Ed.) 1991, "Essential Molecular Biology: A Practical Approach", IRL Press at Oxford University Press, Oxford. Alternatively, polynucleotide variants are obtainable by PCR-based techniques such as mixed oligonucleotide primer- based amplification of DNA, i.e. using degenerated primers against conserved domains of the polypeptides in accordance with the method of the present invention. Conserved domains of the said polypeptide may be identified by a sequence comparison of the nucleic acid sequence of the polynucleotide or the amino acid sequence of the polypeptide. Oligonucleotides suitable as PCR primers as well as suitable PCR conditions are known in the art. As a template, DNA or cDNA from bacteria, fungi, plants or animals may be used. Preferably, such variant polynucleotides comprise or essentially consist of a nucleic acid sequence which is at least 60%, at least 70%, at least 80%, at least 90%, at least 95%, at least 96%, at least 97%, at least 98% or at least 99% identical to the nucleic acid sequences shown in SEQ ID NO: 1, 3, 5, 7, 9, 11, or 13, or a nucleic acid sequence encoding a polypeptide having an amino acid sequence which is at least 60%, at least 70%, at least 80%, at least 90%, at least 95%, at least 96%, at least 97%, at least 98% or at least 99% identical to the amino acid sequences shown in SEQ ID NO: 2, 4, 6, 8, 10, 12, or 14. The term "identical" as used herein refers to sequence identity characterized as determination of the identity of nucleic or amino acid sequences wherein the sequences are aligned so that the highest order match is obtained, and which can be calculated using published techniques or methods codified in computer programs such as, for example, BLASTP, BLASTN, FASTA, Altschul 1990, J Mol Biol 215, 403. The percent identity values are in one aspect calculated over the entire nucleic or amino acid sequence. A series of programs based on a variety of algorithms is available to the skilled worker for comparing different sequences. In this context, the algorithms of Needleman and Wunsch or Smith and Waterman give particularly reliable results. To carry out the sequence alignments, the program PileUp (1987, J Mol Evolution 25, 351; Higgins 1989 CABIOS 5, 151) or the programs Gap and BestFit (Needleman and Wunsch 1970, J Mol Biol 48; 443; Smith and Waterman 1981, Adv Appl Math 2, 482), which are part of the GCG software packet (Genetics Computer Group 1991, 575 Science Drive, Madison, Wisconsin, USA 53711), are to be used. The sequence identity values recited above in percent (%) are to be determined, preferably, using the program GAP over the entire sequence region with the following settings: Gap Weight: 50, Length Weight: 3, Average Match: 10.000 and Average Mismatch: 0.000, which, unless otherwise specified, shall always be used as standard settings for sequence alignments. The percent identity values are, preferably, calculated over the entire nucleic or amino acid sequence using the techniques set forth above.

The polynucleotide of the present invention shall be provided, preferably, either as an isolated polynucleotide (i.e. isolated from its natural context or in purified form) or in genetically modified form. The polynucleotide, preferably, is DNA including cDNA or RNA. The term encompasses single as well as double stranded polynucleotides. Thus, the term "polynucleotide" also encompasses the complementary or reverse complementary strands of the aforementioned specific polynucleotides. Moreover, comprised are also chemically modified polynucleotides including naturally occurring modified polynucleotides such as glycosylated or methylated polynucleotides or artificial modified one such as biotinylated polynucleotides.

The term "medicament" as used herein refers, preferably, to a pharmaceutical composition comprising the biologically active polynucleotide of the present invention as pharmaceutical active compound. Said pharmaceutical composition may be used for human or non-human therapy of various cancers such as astrocytoma, meningioma, bronchial carcinoma, breast carcinoma, ovarian carcinoma, and prostate carcinoma, renal cell carcinoma, hepatic cell carcinoma, melanoma, colon carcinoma, pancreatic carcinoma, gastric cancer, gallbladder cancer, and leukemia in a therapeutically effective dose.

A pharmaceutical composition as used herein comprises the biologically active polynucleotide of the present invention, and optionally one or more pharmaceutically acceptable carrier. The compounds of the present invention can be formulated as pharmaceutically acceptable salts. Acceptable salts comprise acetate, methylester, HCl, sulfate, chloride and the like. The pharmaceutical compositions are, preferably, administered topically or systemically. Suitable routes of administration conventionally used for drug administration are oral, intravenous, or parenteral administration as well as inhalation. However, depending on the nature and mode of action of a drug, the pharmaceutical compositions may be administered by other routes as well. For example, polynucleotide drugs shall be administered in a gene therapy approach by using viral vectors or viruses or liposomes.

Moreover, the drugs comprised by the pharmaceutical composition can be administered in combination with other drugs either in a common pharmaceutical composition or as separated pharmaceutical compositions wherein said separated pharmaceutical compositions may be provided in form of a kit of parts.

The pharmaceutical compositions are, preferably, administered in conventional dosage forms prepared by combining the drugs with standard pharmaceutical carriers according to conventional procedures.

The carrier(s) must be acceptable in the sense of being compatible with the other ingredients of the formulation and being not deleterious to the recipient thereof. The pharmaceutical carrier employed may include a solid, a gel, or a liquid. Exemplary of solid carriers are lactose, terra alba, sucrose, talc, gelatin, agar, pectin, acacia, magnesium stearate, stearic acid and the like. Exemplary of liquid carriers are phosphate buffered saline solution, syrup, oil, water, emulsions, various types of wetting agents, and the like. Similarly, the carrier or diluent may include time delay material well known to the art, such as glyceryl mono-stearate or glyceryl distearate alone or with a wax. Said suitable carriers comprise those mentioned above and others well known in the art, see, e.g., Remington's Pharmaceutical Sciences, Mack Publishing Company, Easton, Pennsylvania.

The pharmaceutical composition may also, preferably, comprise one or more diluent(s). The diluent(s) is/are selected so as not to affect the biological activity of the combination. Examples of such diluents are distilled water, physiological saline, Ringer's solutions, dextrose solution, and Hank's solution. In addition, the pharmaceutical composition or formulation may also include other carriers, adjuvants, or non-toxic, non-therapeutic, non-immunogenic stabilizers and the like.

A therapeutically effective dose refers to an amount of the drug to be used in a pharmaceutical composition of the present invention which prevents, ameliorates or treats the symptoms accompanying a disease or condition referred to in this specification. Therapeutic efficacy and toxicity of the compound can be determined by standard pharmaceutical procedures in cell cultures or experimental animals, e.g., ED50 (the dose therapeutically effective in 50% of the population) and LD50 (the dose lethal to 50% of the population). The dose ratio between therapeutic and toxic effects is the therapeutic index, and it can be expressed as the ratio, LD50/ED50.

The dosage regimen will be determined by the attending physician and other clinical factors. As is well known in the medical arts, dosages for any one patient depends upon many factors, including the patient's size, body surface area, age, the particular compound to be administered, sex, time and route of administration, general health, and other drugs being administered concurrently. Progress can be monitored by periodic assessment.

The pharmaceutical compositions and formulations referred to herein are administered at least once in order to treat or ameliorate various cancers such as astrocytoma, meningioma, bronchial carcinoma, breast carcinoma, ovarian carcinoma, and prostate carcinoma, renal cell carcinoma, hepatic cell carcinoma, melanoma, colon carcinoma, pancreatic carcinoma, gastric cancer, gallbladder cancer, and leukemia. However, the said pharmaceutical compositions may be administered more than one time.

It is to be understood that the formulation of a pharmaceutical composition takes place under GMP standardized conditions or the like in order to ensure quality, pharmaceutical security, and effectiveness of the medicament. Moreover, the medicament shall finally, also preferably, comprise dosage recommendations which shall be indicated in the prescribers or users instructions in order to anticipate dose adjustments depending on the considered recipient.

In accordance with the present invention it was found that full length MLKL (flMLKL) and its orthologs and paralogs are overexpressed in cancer cells. Full length MLKL encoding polynucleotides (flMLKL polynucleotides) are polynucleotides comprising or essentially consisting of a nucleic acid sequence as shown in SEQ ID NO: 1 (Homo sapiens), SEQ ID NO: 7 (Mus musculus), SEQ ID NO: 9 (Rattus norvegicus), or SEQ ID NO: 11, or 13 (Pan troglodytes), or variants thereof as specified above. Moreover, it was found that a further heterologous overexpression of said flMLKL polynucleotides resulted in apoptosis of cancer cells. Moreover, MLKL muteins encoded by polynucleotides having SEQ ID NO: 3, or 5 were found to be capable of inducing apoptosis after heterologous overexpression in cancer cells, too. Thus, MLKL has been identified as an oncogene whose modulation in expression and/or activity allows, advantageously, for developing cancer treatments and diagnostic tests.

Further, the present invention relates to a nucleic acid which specifically binds to a polynucleotide comprising
a) a nucleic acid sequence as shown in SEQ ID NO: 1, 7, 9, 11, or 13,
b) a nucleic acid sequence encoding a polypeptide having an amino acid sequence as shown in SEQ ID NOs: 2, 8, 10, 12, or 14,
c) a nucleic acid sequence which is at least 60% identical to the nucleic acid sequence of (a), or
d) a nucleic acid sequence encoding for a polypeptide having an amino acid sequence which is at least 60% identical to the amino acid sequence of (b)
for use as a medicament, whereby the nucleic acid upon specific binding prevents the generation of a biologically active polypeptide encoded by the said polynucleotide.

"Nucleic acid" as used in accordance with the aforementioned embodiment of the present invention relates to a nucleic acid which specifically binds to a polynucleotide encoding flMLKL polypeptide and variants thereof as specified above. The nucleic acid shall be capable of preventing upon binding to the said polynucleotide the function of a biologically active flMLKL polypeptide. This includes the prevention of the transcription of the gene encoding the flMLKL polypeptide as well as the prevention of the translation of flMLKL encoding transcripts. Moreover, it includes further the induction of degradation of flMLKL encoding transcripts and the inhibition of the activity of the flMLKL polypeptide referred to above.

Thus, a nucleic acid, preferably, encompasses single as well as double stranded nucleotides. The nucleic acid is, more preferably, selected from the group of: siRNA, microRNA, ribozyme, morpholinos, antisense RNA, and triple helix forming oligonucleotides or polynucleotides encoding any of the aforementioned molecules.

The term "siRNA" (small interfering RNA) as used herein relates to a nucleic acid molecule which is a double stranded RNA agent that is complementary to i.e., able to base-pair with, a portion of a target RNA (generally mRNA), i.e. the polynucleotide encoding the flMLKL polypeptide of the present invention being RNA. The siRNA acts to specifically guide enzymes in the host cell to cleave the target RNA. By virtue of the specificity of the siRNA sequence and its homology to the RNA target, siRNA is able to cause cleavage of the target RNA strand, thereby inactivating the target RNA molecule. Preferably, the siRNA which is sufficient to mediate RNAi comprises a nucleic acid sequence comprising an inverted repeat fragment of the target gene and the coding region of the gene of interest (or portion thereof). RNAi (RNA interference) refers to selective intracellular degradation of RNA used to silence expression of a selected target gene, i.e. the polynucleotide encoding flMLKL polypeptide of the present invention. RNAi is a process of sequence-specific, post-transcriptional gene silencing in organisms initiated by double-stranded RNA (dsRNA) that is homologous in sequence to the gene to be silenced. The RNAi technique involves small interfering RNAs (siRNAs) that are complementary to target RNAs (encoding a gene of interest) and specifically destroy the known mRNA, thereby diminishing or abolishing gene expression. RNAi is generally used to silence expression of a gene of interest by targeting mRNA, however, any type of RNA is encompassed by the RNAi methods of the invention. Briefly, the process of RNAi in the cell is initiated by long double stranded RNAs (dsRNAs) being cleaved by a ribonuclease, thus producing siRNA duplexes. The siRNA binds to another intracellular enzyme complex which is thereby activated to target whatever mRNA molecules are homologous (or complementary) to the siRNA sequence. The function of the complex is to target the homologous mRNA molecule through base pairing interactions between one of the siRNA strands and the target mRNA. The mRNA is then cleaved approximately 12 nucleotides from the 3' terminus of the siRNA and degraded. In this manner, specific mRNAs can be targeted and degraded, thereby resulting in a loss of protein expression from the targeted mRNA. A complementary nucleotide sequence as used herein refers to the region on the RNA strand that is complementary to an RNA transcript of a portion of the target gene. The term "dsRNA" refers to RNA having a duplex structure comprising two complementary and anti-parallel nucleic acid strands. Not all nucleotides of a dsRNA necessarily exhibit complete Watson-Crick base pairs; the two RNA strands may be substantially complementary. The RNA strands forming the dsRNA may have the same or a different number of nucleotides, with the maximum number of base pairs being the number of nucleotides in the shortest strand of the dsRNA. Preferably, the dsRNA is no more than 49, more preferably less than 25, and most preferably between 19 and 23, nucleotides in length. dsRNAs of this length are particularly efficient in inhibiting the expression of the target gene using RNAi techniques. dsRNAs are subsequently degraded by a ribonuclease enzyme into short interfering RNAs (siRNAs).

Methods relating to the use of RNAi to silence genes in organisms, including C. elegans, Drosophila, plants, and mammals, are known in the art (see, for example, Fire et al., Nature (1998) 391:806-811; Fire, Trends Genet. 15, 358-363 (1999); Sharp, RNA interference 2001. Genes Dev. 15,485-490 (2001); Hammond et al. Nature Rev. Genet. 2, 1110-1119 (2001); Tuschl, Chem. Biochem. 2, 239-245 (2001); Hamilton et al., Science 286, 950-952 (1999); Hammond et al., Nature 404, 293-296 (2000); Zamore et al., Cell 101, 25-33 (2000); Bernstein et al., Nature 409, 363-366 (2001); Elbashir et al., Genes Dev. 15, 188-200 (2001); WO 0129058; WO 09932619; and Elbashir et al., 2001 Nature 411: 494-498).

RNAi may be used to specifically inhibit expression of the polynucleotides of the present invention in vivo. Accordingly, it may be used for therapeutic approaches for diseases or disorders which are accompanied with an altered expression of the polynucleotides of the present invention, e.g., an enhanced expression or an expression of the polynucleotides at wrong locations. For such therapeutic approaches, expression constructs for siRNA may be introduced into target cells of the host which suffer from altered polynucleotide expression. Accordingly, siRNA may be combined efficiently with the available gene therapy approaches.

Also preferably, a nucleic acid sequence encoding a siRNA comprising a sequence sufficiently complementary to a target gene is operatively linked to an expression control sequence. Thus, the mediation of RNAi to inhibit expression of the target gene can be modulated by said expression control sequence. Preferred expression control sequences are those which can be regulated by a exogenous stimulus, such as the tet operator whose activity can be regulated by tetracycline or heat inducible promoters. Alternatively, an expression control sequence may be used which allows tissue- specific expression of the siRNA.

The complementary regions of the siRNA allow sufficient hybridization of the siRNA to the target RNA and thus mediate RNAi. In mammalian cells, siRNAs are approximately 21-25 nucleotides in length (see Tuschl et al. 1999 and Elbashir et al. 2001). The siRNA sequence needs to be of sufficient length to bring the siRNA and target RNA together through complementary base-pairing interactions. The siRNA used with the Tet expression system of the invention may be of varying lengths. The length of the siRNA is preferably greater than or equal to ten nucleotides and of sufficient length to stably interact with the target RNA; specifically 15-30 nucleotides; more specifically any integer between 15 and 30 nucleotides, most preferably 15, 16, 17, 18, 19, 20, 21, 22, 23, 24, 25, 26, 27, 28, 29, and 30. By "sufficient length" is meant an oligonucleotide of greater than or equal to 15 nucleotides that is of a length great enough to provide the intended function under the expected condition. By "stably interact" is meant interaction of the small interfering RNA with target nucleic acid (e.g., by forming hydrogen bonds with complementary nucleotides in the target under physiological conditions).

Generally, such complementarity is 100% between the siRNA and the RNA target, but can be less if desired, preferably 91%, 92%, 93%, 94%, 95%, 96%, 97%, 98%, or 99%. For example, 19 bases out of 21 bases may be base-paired. In some instances, where selection between various allelic variants is desired, 100% complementary to the target gene is required in order to effectively discern the target sequence from the other allelic sequence. When selecting between allelic targets, choice of length is also an important factor because it is the other factor involved in the percent complementary and the ability to differentiate between allelic differences.

The term "microRNA" (miRNA) as used herein relates to single-stranded RNA molecules of 21-26 nucleotides in length, which regulate gene expression. miRNAs are encoded by genes from whose DNA they are transcribed but miRNAs are not translated into protein (non-coding RNA); instead each primary transcript (a pri-miRNA) is processed into a short stem-loop structure called a pre-miRNA and finally into a functional miRNA. Mature miRNA molecules are partially complementary to one or more messenger RNA (mRNA) molecules, and their main function is to down-regulate gene expression Mature miRNAs can interact with Argonaute to form the RNA-induced silencing complex (RISC) and then guide the RISC to their target mRNAs, most favorably to the 3'-untranslated region (3'UTR). In order for a miRNA to produce functional consequences, its 5'-end 7 to 8 nucleotides must have exact base pairing to the target mRNA and partial complementarity with the rest of the sequence. An miRNA can either inhibit translation or induce degradation of its target mRNA, primarily depending upon the overall degree of complementarity of the binding site, number of binding sites, and the accessibility of the bindings sites (determined by free energy states). The greater the complementarity of the accessible binding sites, the more likely a miRNA degrades its targeted mRNA, and those miRNAs that display imperfect sequence complementarities with target mRNAs primarily result in translational inhibition, see Wang 2008 J Mol Med 86, 771-83.

The term "ribozyme" as used herein relates to a RNA molecule of at least 30 nucleotides in length that catalyzes a chemical reaction, such as the hydrolysis of phosphodiester bonds, or the aminotransferase activity of the ribosome, see Cech 2002, Biochem Soc Trans 30, 1162-6; Cech 1993 Gene 135, 33-6. The said ribozyme will specifically cleave the polynucleotide encoding the flMLKL polypeptide or variants thereof resulting in gene silencing either by mRNA degradation or suppression of translation. The said ribozyme is, preferably, a transacting ribozyme (e.g. hammerhead ribozyme) consisting of a catalytic core, flanked by two arms that share complementarity with the RNA sequence to be cleaved, see Phylactou 1998, Hum Mol Genet 7, 1649-53. Moreover, comprised are synthetic and natural ribozymes.

The term "morpholino" as used herein relates to a molecule sometimes referred to as phosphorodiamidate morpholino oligonucleotide used to modify gene expression. Said Morpholinos are synthetic molecules which are the product of a redesign of natural nucleic acid structure. Usually 25 bases in length, they bind to complementary sequences of RNA by standard nucleic acid base-pairing. Structurally, the difference between Morpholinos and DNA is that while Morpholinos have standard nucleic acid bases, those bases are bound to morpholine rings instead of deoxyribose rings and linked through phosphorodiamidate groups instead of phosphates. Morpholinos are most commonly used as single-stranded oligos, though heteroduplexes of a Morpholino strand and a complementary DNA strand may be used in combination with cationic cytosolic delivery reagents. Morpholino oligomers (oligos) are an antisense technology used to block access of other molecules to specific sequences within nucleic acid. Morpholinos block small (∼25 base) regions of the base-pairing surfaces ofribonucleic acid (RNA). Morpholinos are usually used to knock down gene function. This is achieved by preventing cells from making a targeted protein or by modifying the splicing of pre-mRNA.

The term "antisense RNA" as used herein relates to single-stranded RNA that is complementary to a messenger RNA (mRNA) strand transcribed within a cell. Antisense RNA may be introduced into a cell to inhibit translation of a complementary mRNA by base pairing to it and physically obstructing the translation machinery. This effect is therefore stoichiometric. Preferably, said antisense RNA is complementary to the polynucleotide encoding flMLKL polypeptide or variants thereof and upon base pairing to it obstructing its translation.

The term "triple helix forming oligonucleotide" relates to oligonucleotides which can bind as third strands of DNA in a sequence specific manner in the major groove in polypurine/polypyrimidine stretches in duplex DNA. In one motif, a polypyrimidine oligonucleotide binds in a direction parallel to the purine strand in the duplex. In the alternate purine motif, a polypurine strand binds anti-parallel to the purine strand. The specificity of triple helix formation arises from base triplets (AAT and GGC in the purine motif) formed by hydrogen bonding; mismatches destabilize the triple helix. Triple helix forming oligonucleotides can be designed to bind to sites in gene promoters to block DNA binding proteins and to block transcription both in vitro and in vivo, see Duca 2008 Nucleic Acids Res 36, 5123-38. Preferably, said triple helix forming oligonucleotide is complementary to the polynucleotide encoding flMLKL polypeptide or variants thereof.

It will be understood that the aforementioned preferred nucleic acids are all capable of binding specifically, i.e. without cross reactivity, to the polynucleotide of the invention. This can be achieved, preferably, by including nucleic acid sequences which are complementary to at least a part of the said polynucleotides. The complementary sequences shall be of sufficient length to allow for specific binding. As set forth above, this can be a complementary sequence of at least 15, at least 20, or more nucleotides in length

Furthermore, the present invention relates to an antibody or an RNA-aptamer which specifically recognizes a polypeptide encoded by a flMLKL polynucleotide or a variant thereof for use as a medicament.

The antibody of the present invention shall specifically recognize the flMLKL polypeptide encoded by the polynucleotide of the invention. "Specifically recognize" as used herein means that the antibody of the present invention does not cross react to a significant extent with other epitopes either on the flMLKL polypeptides, or on other polypeptides in general. Specific binding can be tested by various well known techniques including, e.g., competition studies.

Preferably, the antibody of the present invention is a monoclonal antibody, a polyclonal antibody, a single chain antibody, a human or humanized antibody or primatized, chimerized or fragments thereof Also comprised as antibody by the present invention is a bispecific antibody, a synthetic antibody, an antibody fragment, such as Fab, Fv or scFv fragments etc., a phage display antibody or a chemically modified derivative of any of these.

The antibody against the flMLKL polypeptide encoded by the polynucleotide of the invention can be prepared by well known methods using the purified polypeptide encoded by the polynucleotide according to the invention or a suitable fragment derived therefrom as an antigen. A fragment which is suitable as an antigen may be identified by antigenicity determining algorithms well known in the art. Such fragments may be obtained either from the polypeptide encoded by the polynucleotide of the invention by proteolytic digestion or may be a synthetic peptide.

Antibodies or fragments thereof can be obtained by using methods which are described, e.g., in Harlow and Lane "Antibodies, A Laboratory Manual", CSH Press, Cold Spring Harbor, 1988. Monoclonal antibodies can be prepared by the techniques originally described in Köhler and Milstein, Nature 256 (1975), 495, and Galfré, Meth. Enzymol. 73 (1981), 3, which comprise the fusion of mouse myeloma cells to spleen cells derived from immunized mammals.

The antibody can be used, for example, for the immunoprecipitation and immunolocalization of the polypeptide encoded by the polynucleotide of the invention as well as for the monitoring of the presence of said variant polypeptides, for example, in recombinant organisms, and for the identification of compounds interacting with the proteins according to the invention. For example, surface plasmon resonance as employed in the BIAcore system can be used to increase the efficiency of phage antibodies which bind to an epitope of the protein of the invention (Schier, Human Antibodies Hybridomas 7 (1996), 97-105; Malmborg, J. Immunol. Methods 183 (1995), 7-13).

However, the antibody referred to in accordance with the present invention shall be, preferably, capable of reducing or inhibiting the flMLKL activity upon binding to said polypeptide. Antibodies having said desired property can be identified by the assays known in the art and those specified below.

The RNA-aptamer as used herein relates to relatively short (12-30 bases) RNA oligonucleotides, in a specific three-dimensional structure that specifically bind to a target molecule, i.e., the flMLKL polypeptide encoded by the polynucleotide of the present invention and upon binding elicit a biological response, see Ireson 2006, Mol Cancer Ther 12, 2957-62. Said aptamers show high specific binding analogous to that of the antibody to an epitope (see above). Said aptamers are usually created by selecting them from a large random sequence pool, engineered through repeated rounds of in vitro selection or equivalently, SELEX (systematic evolution of ligands by exponential enrichment) to bind to various molecular targets such as small molecules, proteins, nucleic acids, and even cells, tissues and organisms. Said aptamers can be engineered completely in a test tube, are readily produced by chemical synthesis, possess desirable storage properties, and elicit little or no immunogenicity in therapeutic applications. Suitable RNA-aptamers referred to in accordance with the present invention are, preferably, also those capable of reducing or inhibiting the flMLKL activity upon binding to said polypeptide.

The present invention relates to the use of the polynucleotide of the present invention, the nucleic acid of the present invention, or the antibody, or the RNA-aptamer of the present invention for the manufacture of a medicament for the treatment of cancer.

The present invention also relates to a method for diagnosing cancer in a subject comprising
a) determining the amount of a polynucleotide encoding flMLKL polypeptide, or variants thereof, or a polypeptide encoded thereby in a sample of said subject;
b) comparing the amount of polynucleotide or of a polypeptide encoded by it determined in step (a) with a reference amount of the said polynucleotide or polypeptide; and
c) diagnosing cancer in said subject based on the results obtained in step (b).

The term "diagnosing" as used herein refers to assessing the probability according to which a subject is suffering or will suffer from a cancer referred to below in this specification. As will be understood by those skilled in the art, such an assessment is usually not intended to be correct for 100% of the subjects to be diagnosed. The term, however, requires that a statistically significant portion of subjects can be correctly diagnosed to suffer from the cancer. Whether a portion is statistically significant can be determined without further ado by the person skilled in the art using various well known statistic evaluation tools, e.g., determination of confidence intervals, p-value determination, Student's t-test, Mann-Whitney test etc.. Details are found in Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.1, 0.05, 0.01, 0.005, or 0.0001. Preferably, the probability envisaged by the present invention allows that the diagnosis will be correct for at least 60%, at least 70%, at least 80%, or at least 90% of the subjects of a given cohort or population. Diagnosing according to the present invention is preferably done using a diagnostic means. A diagnostic means is any means that allows measuring the level, amount, or concentration of a substance of interest, particularly a polynucleotide encoding flMLKL polypeptide or variants thereof. In a preferred embodiment the antibody or the RNA-aptamer of the present invention can be used as diagnostic means.

Cancer as used in the present invention relates to all forms of malignant neoplasms selected from the group of astrocytoma, meningioma, lung carcinoma, breast carcinoma, ovarian carcinoma, and prostate carcinoma, renal cell carcinoma, hepatic cell carcinoma, melanoma, colon carcinoma, pancreatic carcinoma, gastric cancer, gallbladder cancer, and leukemia.

The term "subject" as used herein, relates to animals, preferably mammals, and more preferably, human beings. In a preferred embodiment of the method of the present invention said subject shall suffer from the aforementioned cancer.

"Determining the amount" of the polynucleotide or polypeptide of the present invention relates to measuring the amount or concentration, preferably semi-quantitatively or quantitatively. Measuring can be done directly or indirectly. Direct measuring relates to measuring the amount or concentration of the polynucleotide or polypeptide based on a signal which is obtained from the polynucleotide or polypeptide itself and the intensity of which directly correlates with the number of molecules of the polynucleotide or polypeptide present in the sample. Such a signal - sometimes referred to herein as intensity signal -may be obtained, e.g., by measuring an intensity value of a specific physical or chemical property of the polynucleotide or polypeptide. Indirect measuring includes measuring of a signal obtained from a secondary component (i.e. a component not being the polynucleotide or polypeptide itself) or a biological read out system, e.g., measurable cellular responses, ligands, labels, or enzymatic reaction products.

In accordance with the present invention, determining the amount of the polynucleotide or polypeptide can be achieved by all known means. Said means comprise immunoassay devices and methods which may utilize labeled molecules in various sandwich, competition, or other assay formats. Said assays will develop a signal which is indicative for the presence or absence of the polynucleotide or polypeptide. Moreover, the signal strength can, preferably, be correlated directly or indirectly (e.g. reverse- proportional) to the amount of the polynucleotide or polypeptide present in a sample. Further suitable methods comprise measuring a physical or chemical property specific for the polynucleotide or polypeptide such as its precise molecular mass or NMR spectrum. Said methods comprise, preferably, biosensors, optical devices coupled to immunoassays, biochips, analytical devices such as mass- spectrometers, NMR- analyzers, or chromatography devices. Further, methods include micro-plate ELISA-based methods, fully-automated or robotic immunoassays, or PCR and hybridization methods. Probes and PCR primers for the nucleic acid of the present invention can be derived from the flMLKL polypeptide encoding polynucleotide and variants thereof.

It will be understood, that, preferably, determining the amount of the flMLKL polypeptide or its variants can be performed using the antibody and/or the RNA-aptamer of the present invention.

The term "comparing" as used herein encompasses comparing the amount of the polynucleotide or polypeptide of the present invention comprised by the sample to be analyzed with an amount of a suitable reference source specified elsewhere in this description. It is to be understood that comparing as used herein refers to a comparison of corresponding parameters or values, e.g., an absolute amount is compared to an absolute reference amount while a concentration is compared to a reference concentration or an intensity signal obtained from a test sample is compared to the same type of intensity signal of a reference sample. The comparison referred to in step (b) of the method of the present invention may be carried out manually or computer assisted. For a computer assisted comparison, the value of the determined amount may be compared to values corresponding to suitable references which are stored in a database by a computer program. The computer program may further evaluate the result of the comparison, i.e. automatically provide the desired assessment in a suitable output format. Based on the comparison of the amount determined in step a) and the reference amount, a cancer is diagnosed. Therefore, the reference amount is to be chosen so that either a difference or a similarity in the compared amounts allows allocation of subjects in groups with a cancer or without.

Accordingly, the term "reference amount" as used herein refers to an amount which allows for diagnosing cancer in a subject suffering from it as referred to above. Accordingly, the reference may be derived from (i) a sample of a subject known to suffer from cancer (ii) a sample of a subject known not to suffer from cancer. Moreover, the reference amount according to the invention may define a threshold amount, whereby an amount of the polynucleotide or polypeptide larger than the threshold shall be indicative for a subject having cancer while an amount of the polynucleotide or polypeptide lower than the threshold amount shall be an indicator for a subject without cancer. In a preferred embodiment in accordance with the method of the present invention said threshold represents the detectable limit (dependent on the technique used). Thus, the presence of the polynucleotide or polypeptide (i.e. an amount of the polynucleotide or polypeptide above the detectable limit as the threshold) will be indicative for cancer in a subject, while the absence will be indicative for a healthy subject with respect to the said cancer.

The reference amount applicable for an individual subj ect may vary depending on various physiological parameters including age, sex, as well as on the means used for the determination of the polypeptide or peptide referred to herein. A suitable reference amount may be determined by the method of the present invention from a reference sample to be analyzed together, i.e. simultaneously or subsequently, with the test sample. A preferred reference amount serving as a threshold may be derived from the upper limit of normal (ULN), i.e. the upper limit of the physiological amount to be found in a population. The ULN for a given population of subjects can be determined by various well known techniques. A suitable technique may be to determine the median of the population for the peptide or polypeptide amounts to be determined in the method of the present invention.

In a preferred embodiment of the method of the present invention, an amount of said polynucleotide or polypeptide larger than said reference amount indicates cancer in said subject and/or wherein an amount of said the polynucleotide or polypeptide lower than said reference amount indicates no cancer.

It is to be understood that the definitions and explanations of the terms made above apply mutatis mutandis for all embodiments described in this specification and in the following except as otherwise indicated.

The present invention refers in principle to the use of the polynucleotide of the present invention for the manufacture of a diagnostic composition for diagnosing cancer.

The term "diagnostic composition" refers to any composition formulated in solid, liquid or gaseous form. Said composition comprises the compound of the invention optionally together with suitable auxiliary compounds such as diluents or carriers or further ingredients. In this context, it is distinguished for the present invention between auxiliary compounds, i.e. compounds which do not contribute to the effects elicited by the compounds of the present invention upon application of the composition for its desired purpose, and further ingredients, i.e. compounds which contribute a further effect or modulate the effect of the compounds of the present invention. Suitable diluents and/or carriers depend on the purpose for which the composition is to be used and the other ingredients. The person skilled in the art can determine such suitable diluents and/or carriers without further ado. Examples of suitable carriers and/or diluents are well known in the art and include saline solutions such as buffers, water, emulsions, such as oil/water emulsions, various types of wetting agents, etc.. Said composition comprises as the compound the antibody of the present invention which specifically recognizes the polypeptide encoded by the polynucleotide of the present invention.

The present invention refers, in principle, to the use of the antibody, or RNA-aptamer which specifically recognizes the polypeptide encoded by a flMLKL polynucleotide or a variant thereof for the manufacture of a diagnostic composition for diagnosing cancer.

The present invention relates to a method for assessing whether a subject suffering from cancer will benefit from a MLKL based anti-cancer treatment comprising the steps of:
a) determining the amount of a polynucleotide encoding flMLKL polypeptide, or variants thereof, or a polypeptide encoded thereby;
b) comparing the amount determined in step (a) with a reference amount; and
c) identifying whether said subject suffering from cancer will benefit from MLKL based anti-cancer treatment based on the results obtained in step (b).

The term "assessing" as used herein refers to evaluating the probability according to which a subject suffering from a cancer referred to above in this specification will benefit from an MLKL based anti-canter treatment. As will be understood by those skilled in the art, such an evaluation is usually not intended to be correct for 100% of the subjects to be assessed. The term, however, requires that a statistically significant portion of subjects suffering from the said cancer can be correctly assessed whether they will benefit from an MLKL based anti-canter treatment or not. Whether a portion is statistically significant can be determined without further ado by the person skilled in the art using various well known statistic evaluation tools, e.g., determination of confidence intervals, p-value determination, Student's t-test, Mann-Whitney test etc.. Details are found in Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.1, 0.05, 0.01, 0.005, or 0.0001. Preferably, the probability envisaged by the present invention allows that the assessment will be correct for at least 60%, at least 70%, at least 80%, or at least 90% of the subjects of a given cohort or population. Assessing according to the present invention is preferably done using a diagnostic means.

Further, the present invention relates to a method for identifying MLKL modulators comprising the steps:
a) contacting a cell expressing the polynucleotide encoding a flMLKL polypeptide with a compound suspected to be a MLKL modulator; and
b) determining cell viability, whereby an increased or decreased cell viability compared to a control cell identifies said modulator.

"MLKL modulator" as used in the present invention relates to compound capable of altering (enhancing or reducing) the biological activity of the MLKL polypeptide.

The term "contacting" as used herein refers to bringing at least two different compounds in physical proximity as to allow physical and/or chemical interaction of said compounds. In accordance with the method of this invention, the said two different compounds are, preferably, a cell expressing the polynucleotide encoding flMLKL polypeptide or variants thereof and a compound suspected to be a MLKL modulator. Contacting as meant herein is carried out under conditions and for a time being sufficient to allow interaction of a cell expressing the said polynucleotide and the said compound suspected to be a MLKL modulator. Said interaction shall result in binding of the said polynucleotide or a polypeptide encoded thereby to the said compound suspected to be a MLKL modulator. As set forth elsewhere herein, said interaction comprises various kinds of binding such as indirect and direct, non-reversible and reversible measures. Suitable conditions are such, which allow for specific interaction of a cell expressing the said polynucleotide and the said compound suspected to be a MLKL modulator. This is well known to the skilled worker and said condition can depend on the cell expressing the said polynucleotide and the said compound suspected to be a MLKL modulator to be applied in the method determined without further ado. Moreover, a time being sufficient to allow interaction can also be determined by the skilled worker without further ado.

"Determining cell viability" as used herein relates to measuring the amount of cells that are living or dead, based on a total cell sample. Measurements for cell viability relate in general to viability/cytotoxicity, proliferation, and apoptosis assays which are performed using different well known techniques such as fluorescence, radioisotopic techniques, colorimetric methods, and ⁵¹Cr-release. Comprised in accordance with the method of the present invention are assays for viability and cytotoxicity including methods based on using esterase substrates, using nucleic acids stains, measuring oxidation and reduction, assays for cell enumeration, cell proliferation and cell cycle including methods based on nucleic acid probes for cell-cycle analysis, antibodies to proliferation markers and cell-cycle control proteins, endostatin protein and angiostatin protein analysis, assays for apoptosis including methods based on using nucleic acid stains, detecting DNA strand breaks with ChromaTide nucleotides, APO-BrdU TUNEL, using Annexin V conjugates, protease activity, using mitochondrial stains, using free radical probes, using ion indicators, using esterase substrates, measuring the ATP:ADP ratio. In a preferred embodiment of the method of the present invention said cell viability is, preferably, determined using a Cell Titer Blue assay (Promega), WST-1, Cell Death Detection assay (Roche) or BrDU Flow assay by FACS analyses.

"Control cell" as used herein relates to a cell which has not been brought into contact with the compound suspected to be MLKL modulator a cell that lacks or has substantially reduced MLKL polynucleotide or polypeptide levels.

The present invention relates also to a method for identifying MLKL modulators comprising the steps:
a) contacting a polypeptide encoded by the flMLKL polynucleotide or a variant thereof with a compound suspected to be a MLKL modulator; and
b) determining kinase activity the flMLKL polypeptide, whereby an increased or decreased kinase activity compared to a control identifies said modulator.

"Determining kinase activity" as used herein relates to the measurement of phosphorylation of a substrate of the flMLKL polypeptide. An increase or decrease of phosphorylation and, thus, of kinase activity can be, preferably, determined via the amount of ATP substrate compared to a control. If the flMLKL retains kinase activity, the amount of ATP substrate will decrease similar as in the case of the control. However, if the flMLKL kinase activity is reduced or absent due to the presence of a flMLKL inhibitor, the amount of ATP will not or only less significantly decrease compared to the initial amount of substrate ATP and will be increased relative to the control. A modulator having activating potential for the flMLKL kinase activity will result in a significant decrease of the ATP amount in comparison to a control. The aforementioned method can be preferably carried out in vitro, e.g. by using purified flMLKL.

A control relates to a flMLKL polypeptide which has not been contacted to the compound suspected to be an MLKL modulator.

Moreover, the present invention relates to a method for identifying MLKL modulators comprising the steps:
a) contacting a polypeptide encoded by the flMLKL polynucleotide or a variant thereof and a RIPK3 or ANKRD7 polypeptide with a compound suspected to be a MLKL modulator under conditions which allow for binding of the polypeptide to a RIPK3 or ANKRD7 polypeptide; and
b) determining binding of polypeptide encoded by the flMLKL polynucleotide or a variant thereof to a RIPK3 or ANKRD7 polypeptide, whereby an increase or decrease of binding compared to a control is indicative for a compound being a modulator.

"RIPK3 polypeptide" as used herein relates to a receptor-interacting serine/threonine-protein kinase 3 or a variant thereof or a fragment capable of being bound by flMLKL. Preferably, said polypeptide has an amino acid sequence having SEQ ID NO: 20 or is encoded by a polynucleotide having SEQ ID NO: 19.

"ANKRD7 polypeptide" as used herein relates to an Ankyrin repeat domain 7 or a variant thereof or a fragment capable of being bound by flMLKL. Preferably, said polypeptide has an amino acid sequence having SEQ ID NO: 15, 17, or is encoded by a polynucleotide having SEQ ID NO: 16, or 18.

In a preferred embodiment in accordance with the method of the present invention the RIPK3 or ANKRD7 polypeptide is labeled with a tag such as a fluorophor.

"Determining binding" as used herein relates to affinity and specificity of a protein-protein interaction measured with analytical methods well known in the art such ELISA, Radioimmunoassays, FRET, BRET, or the yeast two hybrid system for quantitatively determining binding parameters such as e.g., dissociation, or thermodynamic constant. Preferably, said binding is determined using a competitive binding in which two analytes, an unlabeled and a labeled analyte compete for sites to bind to a specific protein, i.e, the said polypeptide encoded by the polynucleotide. An analyte is a substance that is undergoing analysis or is being measured. One of the two analytes in accordance with the present invention is, preferably, the RIPK3 or ANKRD7 polypeptide and the second analyte is the compound suspected to be a MLKL modulator.

A control as used herein refers to the binding observed for the said polypeptide encoded by the flMLKL polynucleotide or a variant thereof and a RIPK3 or ANKRD7 polypeptide which has not been contacted to the compound suspected to be an MLKL modulator.

In a preferred embodiment of the three aforementioned methods, said modulators can act as an agonist, i.e. a compound which increases the MLKL activity, or as an antagonist i.e. a compound which decreases the MLKL activity. The said increase or decrease of MLKL activity is, preferably, statistically significant.

Furthermore, the present invention relates to a method for the manufacture of medicament comprising the steps of the aforementioned methods and a further step of formulating the modulator as a medicament.

All references cited in this specification are herewith incorporated by reference with respect to their entire disclosure content and the disclosure content specifically mentioned in this specification.

The figures show:

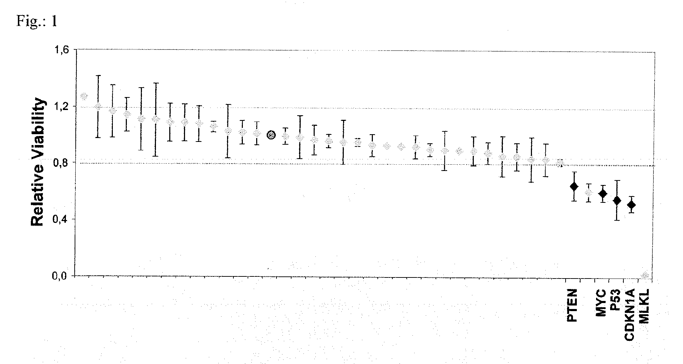

**Figure 1****:** Viability assay after overexpression of candidate genes and controls in U373-MG glioblastoma cells. 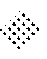 Candidate genes,◆ control genes, 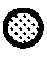 empty vector control. 40 different candidate genes for glioblastoma as well as control genes were stably integrated into isogenetic and inducible U373-MG cells. Cell viability were determined by Cell Titer Blue Assay (Promega) on recombinants treated with (+) or without doxycycline (-) over 5 days. The graph below shows the Percentages ratio of viability of Dox - / Dox + recombinants, normalized to the empty vector control. Error bars are SEM. The blue line marks genes, which display ≥ or ≤ 20% deviation to empty vector control. This screen identifies the novel candidate MLKL which shows the strongest effect on glioblastoma cell viability.

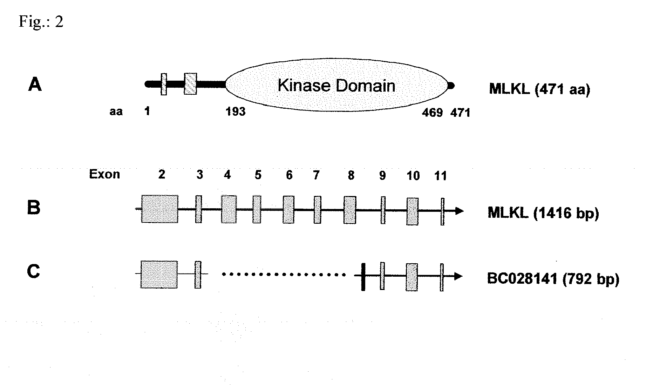

**Figure 2****:** MLKL transcript variants. (A) Protein structure of long variant MLKL 1416, (B) Exon ( 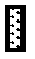 ) structure of long variant, (C) Exon structure ( 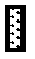 ) of short variant, corresponding to cDNA clone BC028141. BC028141 additionally contains a 30 bp intron sequence ( 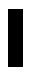 ). Coiled coil domain ( 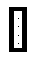 ).

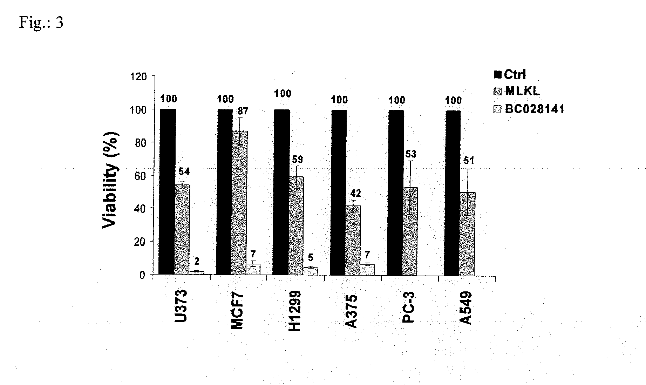

**Figure 3****:** Suppression of viability subsequent to overexpression of MLKL long variant and BC028141, respectively, in U373-MG (glioblastoma), MCF-7 (breast cancer), H1299 (NSCLC), and A375 (melanoma) cell lines. For PC-3 (prostate cancer) and A549 (NSCLC), only the long MLKL-variant was overexpressed and evoked a substantial reduction of viability. Up to now, for PC3 and A549, viable stable cell lines for BC028141 could not be obtained probably due to the strong killing effects of this variant. Cell viability was determined by Cell Titer Blue Assay (Promega) on recombinants treated with or without doxycycline over 5 days. The bar graphs illustrate the percentage viability normalized to empty vector control. Error bars are SEM.

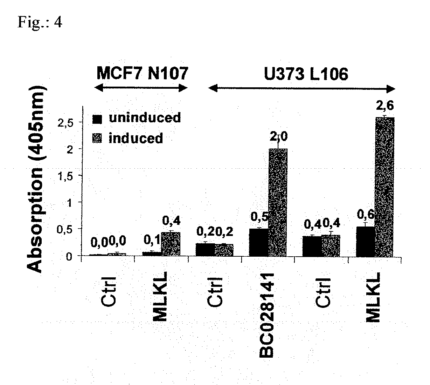

**Figure 4****:** Overexpression of MLKL long variant and BC028141 induces apoptosis in MCF-7 (breast cancer) and U373-MG (glioblastoma) cell lines, respectively. The stable recombinants were seeded in 12-well plates. 24h after induction of the expression, the apoptosis was measured by Cell Death Detection Assay (Roche) and the data were plotted as elevated absorption. Error bars are SEM.

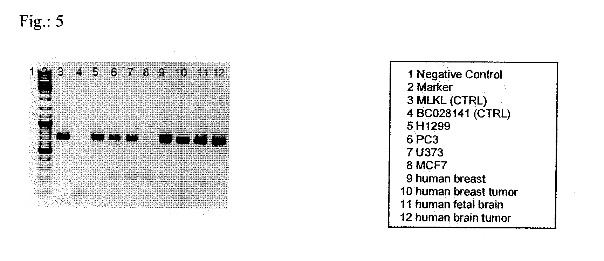

**Figure 5****:** Reverse transcription-polymerase chain reaction (RT-PCR) analysis utilizing primers located in exons 3 and 9 (see Fig.: 2) in order to determine the expression patterns of MLKL variants in different tissues. MLKL long variant appears to be predominantly expressed in the tissues analysed.

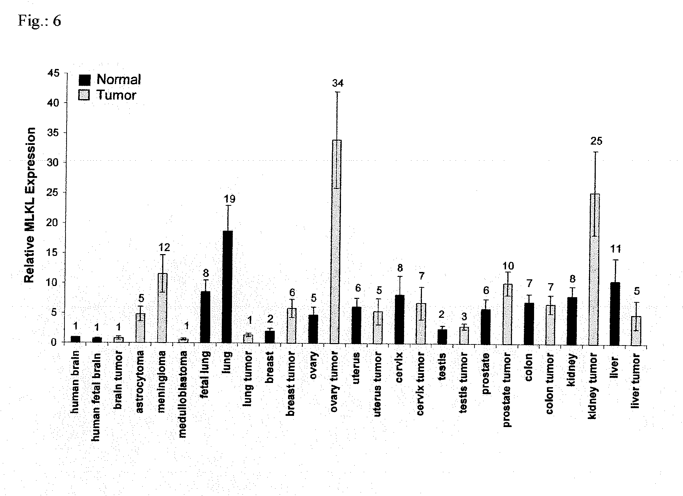

**Figure 6****:** Quantitative RT-PCR analysis of MLKL expression in normal (black) vs. cancer (grey) tissues. Data were normalized to GAPDH and ACTB housekeeping genes. Error bars are SEM.

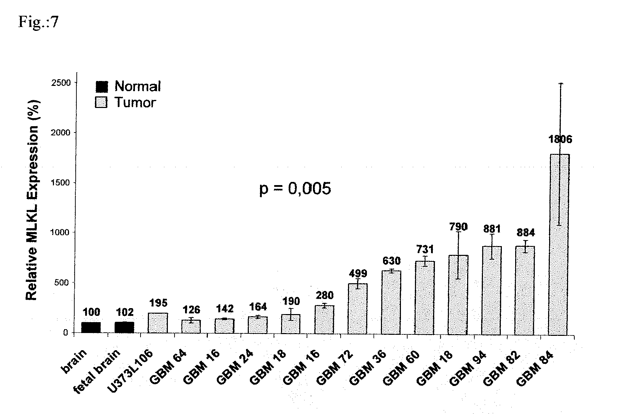

**Figure 7****:** MLKL mRNA is upregulated in glioblastoma (GBM - black) vs. normal brain (grey) tissue samples. The bar graphs illustrate the GAPDH- and ACTB-normalized MLKL mRNA levels in patient samples relative to those in the normal brain tissues. Error bars are SEM.

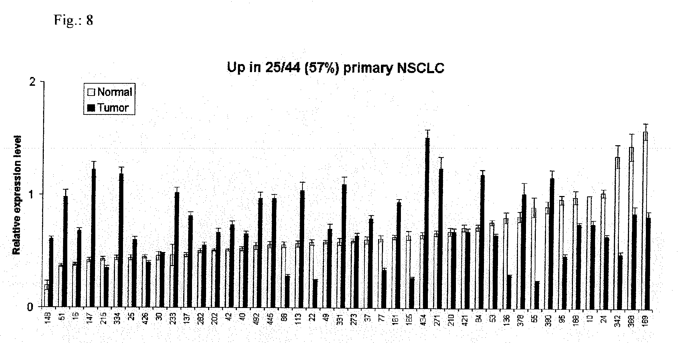

**Figure 8****:** MLKL is upregulated in primary non-small cell lung cancer (NSCLC) cases. The relative expression level represent the ESD- and POLR2A-normalized MLKL mRNA levels in individual tumor tissues (Black columns) relative to those in the respective matched normal tissues from the same patient (grey columns).

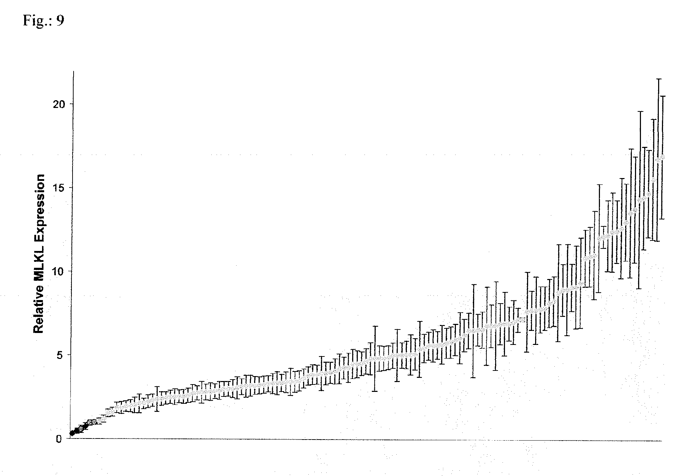

**Figure 9****:** Upregulation of MLKL mRNA in primary breast cancer ( 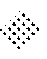 ) compared to normal tissues ( 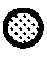 ) and cell lines MCF-10a, MCF-12a (mammary epithelial cell lines) and MCF-7 (breast cancer cell line) (◆). The graph shows the GAPDH- and ACTB-normalized levels of MLKL mRNA by quantitative RT-PCR. Error bars are SEM.

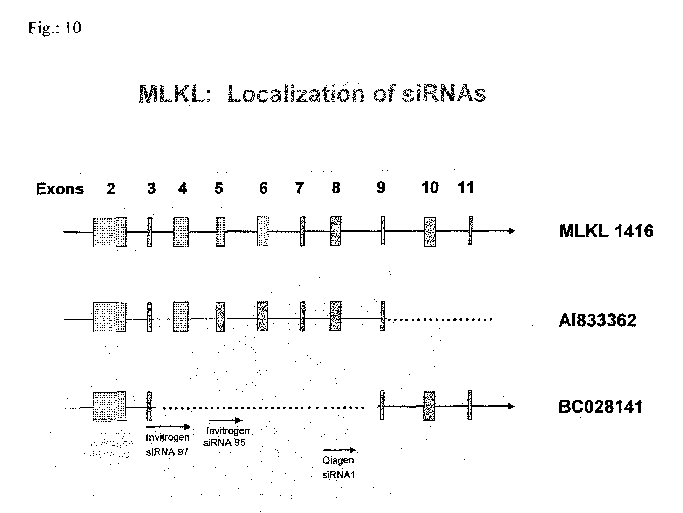

**Figure 10****:** Localization of the MLKL siRNAs.

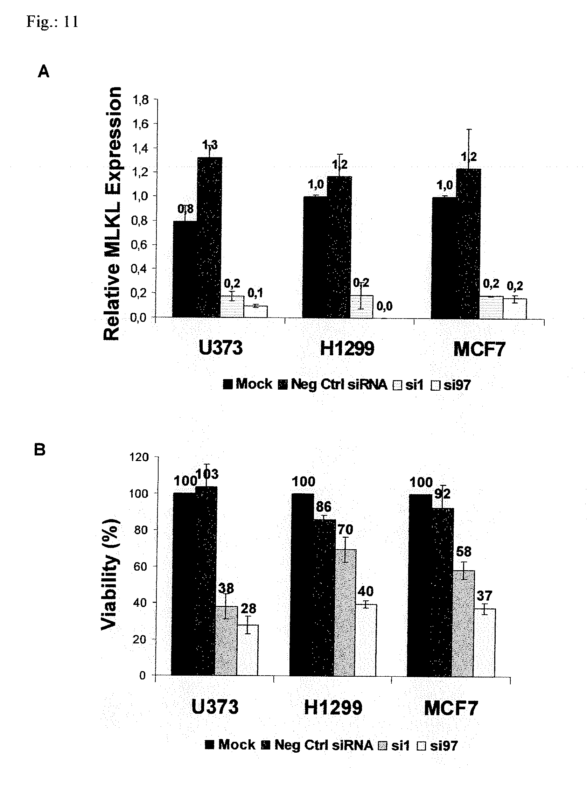

**Figure 11****:** Knock-down of MLKL in U373-MG (glioblastoma), H1299 (NSCLC) and MCF-7 (breast cancer) cell lines. (A) Quantitative RT-PCR-Analysis of MLKL Expression 96 h after siRNA/mock transfection. The graph show the GAPDH and ACTB-mRNA normalized levels of MLKL mRNA in the siMLKL / scrambled siRNA transfected cells expressed as relative level of MLKL mRNA in the mock-transfected cells. (B) Viability of cell lines 96 h after siRNA/mock transfection. Cell Viability was determined by Cell Titer Blue Assay (Promega) at day 0, 2, 3 and 4 after transfection.

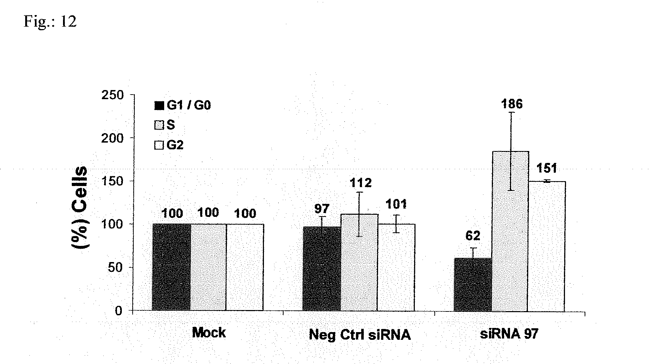

**Figure 12****:** Effect of siRNA MLKL on U373-MG cell cycle progression. One day before transfection, U373-MG cells were plated onto a 6-well plate at 8x10⁴ cells per well. The cells were then transfected by incubation with the respective siRNAs and RNAiMaxx (Invitrogen) as mock-control according to the manufacturer's instructions. 48h later cells were cultured in the presence of 20µM Dif-3 for 24h to induce G1 cell cycle arrest. Cells were then labeled with 7-AAD and analyzed by using a FACScan flow cytometer (Becton Dickinson). Data are representative cell cycle distribution profiles obtained from the mock- and siMLKL 97 transfected cells. MLKL inhibits cell cycle by causing S/G2-Phase arrest.

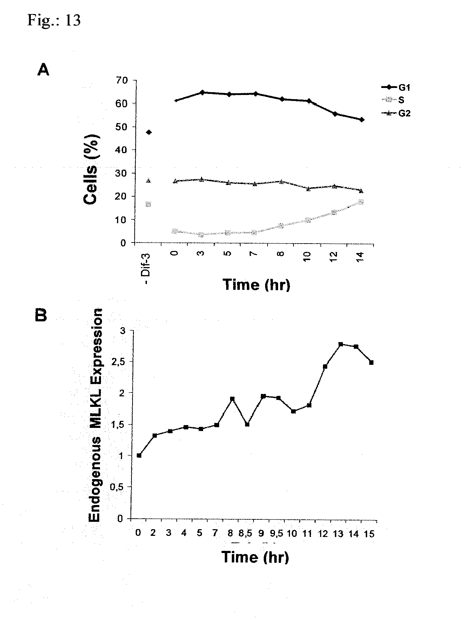

**Figure 13****:** Determination of endogenous MLKL-expression in the course of the cell cycle. (A) Quantitative RT-PCR-Analysis of MLKL in U373-MG cells over 15 h. (B) Distribution of U373-MG cells in the cell cycle phases over 14 h after synchronization in G1-phase with Dif-3.
   Cells were synchronized in G1 by a 24 h-treatment with Dif-3, and were then released into culture media without Dif-3. At the indicated time points, cells were harvested for FACS analysis of cell cycle progression (B), and for total RNA-preparation (A). MLKL expression was determined by TaqMan PCR analysis of reverse-transcribed cDNA.

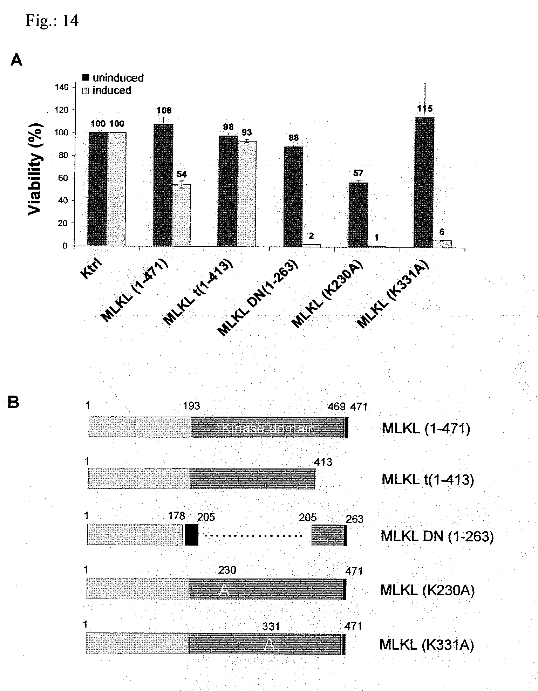

**Figure 14:** (A) Cell viability of U373-MG cells after 5 days of overexpression of different MLKL-constructs compared to cells with empty vector control. MLKL (1-471) = full length variant, MLKL t(1-413) = C-terminal deleted variant, MLKL DN(1-263) = BC028141, MLKL (K230A) and MLKL (K331A) = mutated ATP-, respective nucleotide-binding site. Cell viability was determined by Cell Titer Blue Assay (Promega) on recombinants treated with or without doxycycline over 5 days. The error bars are SEM. (B) Schematic diagrams of the different MLKL constructs used in (A). Numbers indicate the position of amino acid in MLKL.

The following examples merely illustrate the invention and should not be construed, whatsoever, as limiting the scope of the invention.

### Example 1: Cell culture and generation of stably-transfected recombinants

For an inducible overexpression of candidate genes, a FLP-In™ T-REx™-System (Invitrogen) based vector system was used. By Flp recombinase-mediated integration isogenic, stable and inducible expression cell lines were generated. This system was modified and established by the Department of Molecular genome analysis, DKFZ Heidelberg (Blaich S and Wittig R, in preparation). With a conventional procedure for the generation of stable cell lines (tranfection with a parental vector, selection with G418, subcloning and characterization of the expression characteristics), a host cell line containing an integrated FRT site had been established. This cell line was then used as a host to generate a doxycycline-inducible candidate gene expression cell line by cotransfecting the expression vector containing the gene of interest and the Flp recombinase expression plasmid, pOG44 (Invitrogen).
All cell lines were grown as a monolayer in appropriate media, supplemented with 10% fetal bovine serum, 2 mM glutamine, 100 U/ml penicillin and streptomycin as follows: DMEM for U373-MG, MCF7, and A549 cells; RPMI medium for PC3, and A375; RPMI Hepes for H1299.
The expression plasmids were co-transfected with a Flp-recombinase expression vector (pOG44) into cells by GeneJammer reagent (Stratagene) or by the nucleofector device (Lonza) according to the manufacturer's instructions. After 48 h cells were cultured in the selection medium (hygromycin B; Invitrogen) as follows: 90 µg/ml for MCF7 (breast cancer), 300 µg/ml for U373-MG (glioblastoma) and A375 (melanoma), 400 µg/ml for H1299 (lung cancer), 500 µg/ml for A549 (lung cancer) and 70 µg/ml for PC3 (prostate cancer). 3-4 weeks after transfection remaining colonies were picked, expanded and used for cell viability assays. The inducible overexpression of the candidate genes was checked by quantitative RT-PCR (see Example 7).

### Example 2: Viability Screen

For an initial viability screen, 40 different candidate genes for glioblastoma as well as control genes were stably integrated by site specific recombination into isogenic and inducible U373-MG cells. Cell viability was determined by Cell Titer Blue Assay (Promega) on recombinants treated with or without doxycycline over 5 days. This screen recovered MLKL (Accession no. BC028141) as a gene killing brain cancer cells even stronger than known tumor suppressors (CDKN1A, PTEN, TP53) or oncogenes (MYC) did (see Fig.1). New annotations in the database NCBI unfold a longer 1416 bp open reading frame for MLKL (SEQ ID No. 1 and 2), whereas the so far used shorter variant bears 792 bp, and a major part of the putative kinase domain is absent (see Fig. 2). Therefore, the longer annotated MLKL full length variant was cloned (see Example 4 and SEQ ID No. 1) and analyzed for its effect on cell viability (see Table in Example 3). Compared to the short variant, the long variant showed also a strong but a weaker effect on cell viability.

### Example 3: Viability Assay

Recombinants treated with or without doxycycline were seeded in 96-wells in triplicates as follows: 500 cells (A375), 1000 cells (U373-MG, PC-3), respective 2000 cells (MCF7, H1299, A549). Cell viability was measured on day 0, 3 and 5 by Cell Titer Blue Assay (Promega) according to manufacturer's instructions. This assay is based on the conversion of resazurin to the fluorescent resorufin product by viable cells. The fluorescence produced is proportional to the number of metabolically active, viable cells in the population. The fluorescent signal was read using a fluorescence plate reader (Fusion, Packard Biosciences). For Data analysis, the mean values of the triplicates for each day were calculated and normalized to the values of day 0 as well as to the empty vector control. Cell Viability assays were performed in at least three independent experiments.
The short and the long MLKL variant exerted cancer cell killing effects upon expression in all cancer types tested (brain cancer: U373-MG; breast cancer: MCF7; lung cancer: H1299; A549; melanoma: A375, prostate cancer: PC3) with the short variant exerting stronger effects compared to SEQ ID No. 1 (long MLKL variant; see Fig. 3).

The following table summarizes the effects on cell viability after inducible overexpression of different MLKL variants in different stable tumor cell lines:

| Cell line | MLKL (1-263) | MLKL (1-471) | MLKLK230A | MLKL K331A | MLKL t(1-413) |
|---|---|---|---|---|---|
| | short variant | full length | | | |
| | AC: BC028141 | SEQ ID No. 1 | SEQ ID No. 3 | SEQ ID No. 5 | AC:A1833362 |
| U373-MG | ++ | + | ++ | ++ | - |
| MCF7 | ++ | + | ++ | ++ | - |
| H1299 | ++ | + | n.t. | n.t. | n.t. |
| A549 | n.t. | + | n.t. | n.t. | n.t. |
| A375 | ++ | + | n.t. | n.t. | n.t. |
| PC3 | n.t. | + | n.t. | n.t. | n.t. |

| | | | | | |
|---|---|---|---|---|---|
| ++ strong effect on cell viability + effect on cell viability - no effect on cell viability n.t. not tested | | | | | |

### Example 4: Construction of full length and mutant MLKL-variants

The cDNA encoding the full length human MLKL (SEQ ID No. 1) was cloned from the cDNA clones BC028141 and A1833362 by using a modifying PCR and restriction digest. The generated 1416 bp full length MLKL was then amplified by Gateway compatible PCR primers and was cloned into the recombination based vector system as described in example 1.

The mutant MLKL K230A (SEQ ID No. 3) or MLKL K331A (SEQ ID No. 5) was performed using the QuikChange® Site-Directed Mutagenesis Kit (Stratagene). The expression vector with the full length coding region of MLKL was used as a template in a PCR with the following primer pairs containing the respective point mutation:

| | |
|---|---|
| K230A sense | CCACAGAGCTCCAGTGGCCATAGCAGTATTCAAAAAACTCCA |
| K230A_antisense | TGGAGTTTTTTGAATACTGCTATGGCCACTGGAGCTCTGTGG |
| K331A sense | AGCACCTGAACTCCACGGAGCAATCAGAAGCTCAAACTTC |
| K331A_antisense | GAAGTTTGAGCTTCTGATTGCTCCGTGGAGTTCAGGTGCT |

Ten nanograms of plasmid DNA and 125 ng of each primer were used according to the QuikChange® kit. All the point mutants were further verified by DNA sequencing.

MLKL K230A (SEQ ID No. 3) encodes a kinase domain mutant in the ATP binding site, wherein Lysine at position 230 was changed to Alanine. MLKL K331A (SEQ ID No. 5) encodes a kinase domain mutant in the nucleotide-binding site, wherein Lysine at position 331 was changed to Alanine.

All cDNAs encoding the full length respective mutated human MLKL, were cloned into a Gateway ® Technology (Invitrogen) compatible expression vector, which was modified by Dr. S. Blaich and Dr. R. Wittig (DKFZ Heidelberg) (see Example 1).
Both mutant variants exerted a similar strong effect on cell viability as the short MLKL variant did (Fig. 14). Because mutations of amino acids critical for the putative kinase function exert such strong functional effects, this evidences that MLKL acts as a protein kinase. Further, the strong killing effect on cancer cells supports that non-functional variants of MLKL, including the short variant (BC028141 = MLKL DN(1-263)), exert dominant negative effects.

### Example 5: RT-PCR analysis utilizing primers located in MLKL exons 3 and 9

Reverse transcribed mRNA from different tissues or cell lines (see Example 7 and Fig.: 2) was used as template for determination of existing MLKL-variants by PCR.
The PCR was performed with 1x PCR buffer (Applied Biosystems), 200 µM dNTP's, 200 µM each 5' and 3'primer, 2,5 unit Taq polymerase (Applied Biosystems). The following primer sequences were used:

| | |
|---|---|
| MLKL_Exon3 | 5'-AAGCTTCACTGAGACGATTAGA-3' |
| MLKL_Exon 9 | 5'-GATTTCCCAGAGGACGATTC-3' |

The MLKL full length variant (SEQ ID No. 1) as well as the short variant BC028141 served as positive control. PCR amplification was performed using the following program: 95°C for 5 min, 95°C for 30 s, 55 °C for 30 s, and 72°C for 2 min, followed by a final extension of 10 min at 72°C. MLKL was amplified for 35 cycles. PCR products were separated in 1,5 % agarose gels, stained with ethidium bromide and analyzed via UV-light.
The length of the PCR fragment was 84 bp for the short variant (BC028141) and 739 bp for the MLKL full length variant. The long variant (SEQ ID No. 1) was identified as the only detectable or most abundant form in normal tissues and cancer cell lines.

### Example 6: Apoptosis Assay

To document that the effects of short/long MLKL overexpression on cell viability reflected apoptosis, an apoptosis assay was done. Therefore, the stable recombinants were seeded in 12-well plates. 24 h after induction of the expression, the apoptosis was measured by Cell Death Detection ELISA ^{PLUS} (Roche) according to manufacturer's protocol. This kit measures the enrichment of histone-complexed DNA fragments (mono and oligonucleosomes) in the cytoplasm of apoptotic cells and thus relative quantification of apoptosis.
The following table summarizes the detected apoptosis after overexpression of MLKL respectively silencing of MLKL in different cell lines.

| Cell line | MLKL (1-263) | MLKL (1-471) | MLKL K230A |
|---|---|---|---|
| | short variant | full length | |
| | BC028141 | | |
| | AC: BC028141 | SEQ ID No. 1 | SEQ ID No. 3 |
| U373-MG | ++ | ++ | ++ |
| MCF7 | ++ | (+) | n.t. |

| | | | |
|---|---|---|---|
| ++ strong apoptosis (+) weak apoptosis - no or only very weak apoptosis n.t. not tested | | | |

The so far described data suggested that MLKL could act as an oncogene, which overexpression induces apoptosis in cancer cells. This is a commonly observed phenomenon (see for example MYC in Fig. 1, which is a known oncogene having this effect (Nilsson and Cleveland, 2003; Ponzielli et al., 2005). The short variant's effects may base on a dominant negative effect, i. e. on inhibiting the function of the naturally more prevalent large variant (SEQ ID No. 1). If so, one would expect: i) that tumors show hyperactivity of the long variant, and ii) that an inhibition of MLKL would likewise exert a cancer cell-killing effect.

### Example 7: Quantitative Taq Man PCR analysis of MLKL expression

For measuring the level of MLKL expression, total RNA was isolated from different cell lines, respective from normal or tumor tissue. First-strand cDNA was made e.g. using the Superscript III Reverse transcriptase kit (Invitrogen) according to manufacturer's protocol. Total MLKL mRNA was measured by means of an ABI PRISM 7900HT sequence detection system (Applied Biosystems) during 40 cycles. In general, Actin, beta and glyceraldehyde-3-phosphate dehydrogenase were used for normalization using predeveloped TaqMan Assay reagent (ACTB: Hs99999903_m1; GAPDH: Hs99999905_m1, Applied Biosystems). The annealing temperature for the amplification of total MLKL was 60°C. The comparative C_{T} method was used to represent the relative expression level of MLKL with respect to a calibrator sample (uninduced empty vector control, respective normal tissue). The level of MLKL in patient tumor samples was then plotted relative to those in the normal tissues (Figures 6, 7 and 9).
For the primary non-small cell lung cancer (NSCLC) cases in Figure 8, the relative MLKL expression level represent the esterase D/formylglutathione hydrolase- (ESD: Hs00382661_m1, Applied Biosystems) and polymerase (RNA) II polypeptide A, (POLR2A: Hs00172187_m1, Applied Biosystems) -normalized MLKL mRNA levels in individual tumor tissues relative to those in the respective matched normal tissues from the same patient.
The quantitative RT-PCR analyses of different tumor types demonstrated an up-regulation of MLKL compared to normal tissues for 6 out of 12 cancer types (brain, breast, ovarian, testis, prostate, and kidney cancer; see Fig. 6). In addition, MLKL is up-regulated in 12/12 (100%) of the Glioblastoma, in 26 / 46 (61%) of the lung tumors, and in 116/116 (100%) of the breast tumors (Fig. 7-9).

### Example 8: RNA interference studies

For MLKL knockdown, cells plated in 6-wells were transfected with either a MLKL-specific siRNA or a negative control siRNA with no known homology to mammalian genes using RNAiMaxx (Invitrogen) in Opti-MEM reduced serum medium (Invitrogen). The following siRNAs were used (see Fig. 10):
Stealth™ Select RNAi (Invitrogen):
   HSS136795 (siRNA MLKL_95)
   HSS136796 (siRNA MLKL_96)
   HSS136797 (siRNA MLKL_97)
HS_FLJ34389_1_HP siRNA (siMLKL_1) (Qiagen)
AllStars Negativ Control siRNA AF488 (Qiagen)

After 24 h of siRNA incubation, cultures were re-fed with the medium and conditions indicated for each experiment, and then harvested at various time points.
Harvested cells were either subjected to cell viability assays to determine the effect of MLKL knock-down on tumor growth or were lysed for total RNA preparation for qRT-PCR of MLKL-expression.The silencing efficiency of MLKL siRNAs were verified by quantitative TaqMan PCR analysis (see example 7). The levels of MLKL mRNA in the siMLKL/scrambled siRNA transfected cells were normalized to GAPDH and ACTB-mRNA, and were plotted as expressed level of MLKL mRNA relative to the mock-transfected cells (see Fig. 11A).

For measuring the effects on cell viability after knock-down of MLKL, cells were seeded six-fold in 96-wells for transfection with siRNAs and fresh media were provided 24 h after. Cell viability was determined by Cell Titer Blue Assay (Promega) 48 h, 72 h, 96 h and 120 h after transfection as described in Example 3. The values were normalized to the mock-control. siRNAs resulting in knock-down of MLKL exerted killing effects on brain, lung, and breast cancer cells (see Fig. 11 B).

### Example 9: Measuring the effect of siRNA MLKL on U373-MG progression by cell cycle analysis

One day before transfection, U373-MG cells were plated onto a 6-well plate at 8x10⁴ cells per well. The cells were then transfected by incubation with the respective siRNAs and RNAiMaxx (Invitrogen) as mock-control according to the manufacturer's instructions. 48h later cells were synchronized in G1 by a 24 h-treatment with 20µM Dif-3, and were then released into culture media without Dif-3. Trypsinized cells were collected and pelleted at 1000 rpm (5 min, 4°C). Cells were fixed in ice-cold MeOH overnight and resuspended in 500µl each 7-AAD-Staining solution (5µg/ml 7-AAD in phosphate buffered saline). Cells were analyzed on a FACScan flow cytometer (Becton Dickinson) and analyzed using Cell Quest Pro software and Win MDI. Data in figure 12 are representative cell cycle distribution profiles obtained from the mock- and siMLKL 97 transfected cells. Opposite to the apoptotic effect induced by over-expression, the knock-down of MLKL did not induce apoptosis, but inhibited the progression through the cell cycle, resulting in an accumulation in the S and G2 phase (see Fig. 12).

### Example 10: Determination the endogenous MLKL-expression in the course of cell cycle

U373-MG cells were synchronized in G1 by a 24 h-treatment with Dif-3, and were then released into culture media without Dif-3. At the indicated time points, cells were harvested for FACS analysis of cell cycle progression (see Fig. 13 B), and for total RNA-preparation. MLKL expression was determined by TaqMan PCR analysis of reverse-transcribed cDNA (see Fig. 13 A). Cell cycle-specific expression levels of MLKL peaking in the S-Phase were consistent with the accumulation in the S and G2 phase after knock-down of MLKL (see Example 9).

## Claims

1. A polynucleotide comprising a nucleic acid sequence selected from the group consisting of
a) a nucleic acid sequence as shown in SEQ ID NOs: 1, 3, 5, 7, 9, 11, or 13,
b) a nucleic acid sequence encoding a polypeptide having an amino acid sequence as shown in SEQ ID NOs: 2, 4, 6, 8 10, 12, or 14,
c) a nucleic acid sequence which is at least 60% identical to the nucleic acid sequence of (a), and
d) a nucleic acid sequence encoding for a polypeptide having an amino acid sequence which is at least 60% identical to the amino acid sequence of (b) for use as a medicament.

2. The polynucleotide of claim 1, wherein said polynucleotide is DNA or RNA.

3. A nucleic acid which specifically binds to a polynucleotide comprising
a) a nucleic acid sequence as shown in SEQ ID NO: 1, 7, 9, 11, or 13,
b) a nucleic acid sequence encoding a polypeptide having an amino acid sequence as shown in SEQ ID NOs: 2, 8, 10, 12, or 14,
c) a nucleic acid sequence which is at least 60% identical to the nucleic acid sequence of (a), or
d) a nucleic acid sequence encoding for a polypeptide having an amino acid sequence which is at least 60% identical to the amino acid sequence of (b) for use as a medicament, whereby the nucleic acid upon specific binding prevents the generation of a biologically active polypeptide encoded by the said polynucleotide.

4. The nucleic acid of claim 3, wherein said nucleic acid is selected from the group of:
siRNA, microRNA, ribozyme, morpholinos, antisense RNA, and triple helix forming oligonucleotides.

5. An antibody or a RNA-aptamer which specifically recognizes a polypeptide encoded by a polynucleotide as specified in claim 3 for use as a medicament.

6. Use of a polynucleotide as specified in claims 1 and 2, a nucleic acid as specified in claim 3 or 4, or an antibody, or a RNA-aptamer as specified in claim 5 for the manufacture of a medicament for the treatment of cancer.

7. The use of claim 6, wherein said cancer is selected from the group of astrocytoma, meningioma, lung carcinoma, breast carcinoma, ovarian carcinoma, and prostate carcinoma, renal cell carcinoma, hepatic cell carcinoma, melanoma, colon carcinoma, pancreatic carcinoma, gastric cancer, gallbladder cancer, and leukemia.

8. A method for diagnosing cancer in a subject comprising
a) determining the amount of the polynucleotide as specified in claim 3 in a sample of said subject;
b) comparing the amount of polynucleotide determined in step (a) with a reference amount of the said polynucleotide; and
c) diagnosing cancer in said subject based on the results obtained in step (b).

9. A method for diagnosing cancer in a subject comprising
a) determining the amount of a polypeptide encoded by a polynucleotide as specified in claim 3 in a sample of said subject;
b) comparing the amount of polypeptide determined in step (a) with a reference amount of the said polypeptide; and
c) diagnosing cancer in said subject based on the results obtained in step (b).

10. Use of the polynucleotide as specified in claim 3 for the manufacture of a diagnostic composition for diagnosing cancer.

11. Use of an antibody or a RNA-aptamer which specifically recognizes the polypeptide encoded by a polynucleotide as specified in claim 3 for the manufacture of a diagnostic composition for diagnosing cancer.

12. A method for assessing whether a subject suffering from cancer will benefit from a MLKL based anti-cancer treatment comprising the steps of:
a) determining the amount of polynucleotide as specified in claim 3;
b) comparing the amount of (a) with a reference amount; and
c) identifying whether said subject suffering from cancer will benefit from MLKL based anti-cancer treatment based on the results obtained in step (b).

13. A method for assessing whether a subject suffering from cancer will benefit from a MLKL based anti-cancer treatment comprising the steps of:
a) determining the amount of a polypeptide encoded by a polynucleotide as specified in claim 3;
b) comparing the amount determined in step (a) with a reference amount; and
c) identifying whether said subject suffering from cancer will benefit from MLKL based anti-cancer treatment based on the results obtained in step (b).

14. The method of any one of claims 8, 9, 12 or 13 or the use of claim 10 or 11, wherein said cancer is selected from the group of astrocytoma, meningioma, bronchial carcinoma, breast carcinoma, ovarian carcinoma, and prostate carcinoma, renal cell carcinoma, hepatic cell carcinoma, melanoma, colon carcinoma, pancreatic carcinoma, gastric cancer, gallbladder cancer, and leukemia.

15. A method for identifying MLKL modulators comprising the steps:
a) contacting a cell expressing the polynucleotide as specified in claim 3 with a compound suspected to be a MLKL modulator; and
b) determining cell viability, whereby an increased or decreased cell viability compared to a control cell identifies said modulator.

16. A method for identifying MLKL modulators comprising the steps:
a) contacting a polypeptide encoded by the polynucleotide as specified in claim 3 with a compound suspected to be a MLKL modulator; and
b) determining kinase activity of the said polypeptide, whereby an increased or decreased kinase activity compared to a control phosphorylation identifies said modulator.

17. A method for identifying MLKL modulators comprising the steps:
a) contacting a polypeptide encoded by the polynucleotide as specified in claim 3 and a RIPK3 or ANKRD7 polypeptide with a compound suspected to be a MLKL modulator under conditions which allow for binding of the polynucleotide as specified in claim 3 to a RIPK3 or ANKRD7 polypeptide; and
b) determining binding of polynucleotide as specified in claim 3 to a RIPK3 or ANKRD7 polypeptide, whereby an increase or decrease of binding compared to a control is indicative for a compound being a modulator.

18. The method of any one of claims 15 to 17, wherein said modulator can act as an agonist or as an antagonist of MLKL.

19. A method for the manufacture of medicament comprising the steps of the method of any one of claims 15 to 18 and a further step of formulating the modulator as a medicament.


SEQUENCE LISTING

<110>  DKFZ Deutsches Krebsforschungszentrum, Stiftung des
       öffentlichen Rechts

<120>  Use of MLKL in cancer therapy

<130>  DK66232EP

<160>  20

<170>  PatentIn version 3.5

<210>  1
<211>  1416
<212>  DNA
<213>  Homo sapiens

<400>  1
   atggaaaatt tgaagcatat tatcaccctt ggccaggtca tccacaaacg gtgtgaagag      60
   atgaaatact gcaagaaaca gtgccggcgc ctgggccacc gcgtcctcgg cctgatcaag     120
   cctctggaga tgctccagga ccaaggaaag aggagcgtgc cctctgagaa gttaaccaca     180
   gccatgaacc gcttcaaggc tgccctggag gaggctaatg gggagataga aaagttcagc     240
   aatagatcca atatctgcag gtttctaaca gcaagccagg acaaaatact cttcaaggac     300
   gtgaacagga agctgagtga tgtctggaag gagctctcgc tgttacttca ggttgagcaa     360
   cgcatgcctg tttcacccat aagccaagga gcgtcctggg cacaggaaga tcagcaggat     420
   gcagacgaag acaggcgagc tttccagatg ctaagaagag ataatgaaaa aatagaagct     480
   tcactgagac gattagaaat caacatgaaa gaaatcaagg aaactttgag gcagtattta     540
   ccaccaaaat gcatgcagga gatcccgcaa gagcaaatca aggagatcaa gaaggagcag     600
   ctttcaggat ccccgtggat tctgctaagg gaaaatgaag tcagcacact ttataaagga     660
   gaataccaca gagctccagt ggccataaaa gtattcaaaa aactccaggc tggcagcatt     720
   gcaatagtga ggcagacttt caataaggag atcaaaacca tgaagaaatt cgaatctccc     780
   aacatcctgc gtatatttgg gatttgcatt gatgaaacag tgactccgcc tcaattctcc     840
   attgtcatgg agtactgtga actcgggacc ctgagggagc tgttggatag ggaaaaagac     900
   ctcacacttg gcaagcgcat ggtcctagtc ctgggggcag cccgaggcct ataccggcta     960
   caccattcag aagcacctga actccacgga aaaatcagaa gctcaaactt cctggtaact    1020
   caaggctacc aagtgaagct tgcaggattt gagttgagga aaacacagac ttccatgagt    1080
   ttgggaacta cgagagaaaa gacagacaga gtcaaatcta cagcatatct ctcacctcag    1140
   gaactggaag atgtatttta tcaatatgat gtaaagtctg aaatatacag ctttggaatc    1200
   gtcctctggg aaatcgccac tggagatatc ccgtttcaag gctgtaattc tgagaagatc    1260
   cgcaagctgg tggctgtgaa gcggcagcag gagccactgg gtgaagactg cccttcagag    1320
   ctgcgggaga tcattgatga gtgccgggcc catgatccct ctgtgcggcc ctctgtggat    1380
   gaaatcttaa agaaactctc cacctttcct aagtag                              1416

<210>  2
<211>  471

<212>  PRT
<213>  Homo sapiens

<400>  2

<210>  3
<211>  1416
<212>  DNA
<213>  artificial

<220>
<223>  MLKL-mutein, K230A

<400>  3
   atggaaaatt tgaagcatat tatcaccctt ggccaggtca tccacaaacg gtgtgaagag      60
   atgaaatact gcaagaaaca gtgccggcgc ctgggccacc gcgtcctcgg cctgatcaag     120
   cctctggaga tgctccagga ccaaggaaag aggagcgtgc cctctgagaa gttaaccaca     180
   gccatgaacc gcttcaaggc tgccctggag gaggctaatg gggagataga aaagttcagc 240
   aatagatcca atatctgcag gtttctaaca gcaagccagg acaaaatact cttcaaggac 300
   gtgaacagga agctgagtga tgtctggaag gagctctcgc tgttacttca ggttgagcaa 360
   cgcatgcctg tttcacccat aagccaagga gcgtcctggg cacaggaaga tcagcaggat 420
   gcagacgaag acaggcgagc tttccagatg ctaagaagag ataatgaaaa aatagaagct 480
   tcactgagac gattagaaat caacatgaaa gaaatcaagg aaactttgag gcagtattta 540
   ccaccaaaat gcatgcagga gatcccgcaa gagcaaatca aggagatcaa gaaggagcag 600
   ctttcaggat ccccgtggat tctgctaagg gaaaatgaag tcagcacact ttataaagga 660
   gaataccaca gagctccagt ggccatagca gtattcaaaa aactccaggc tggcagcatt 720
   gcaatagtga ggcagacttt caataaggag atcaaaacca tgaagaaatt cgaatctccc 780
   aacatcctgc gtatatttgg gatttgcatt gatgaaacag tgactccgcc tcaattctcc 840
   attgtcatgg agtactgtga actcgggacc ctgagggagc tgttggatag ggaaaaagac 900
   ctcacacttg gcaagcgcat ggtcctagtc ctgggggcag cccgaggcct ataccggcta 960
   caccattcag aagcacctga actccacgga aaaatcagaa gctcaaactt cctggtaact 1020
   caaggctacc aagtgaagct tgcaggattt gagttgagga aaacacagac ttccatgagt 1080
   ttgggaacta cgagagaaaa gacagacaga gtcaaatcta cagcatatct ctcacctcag 1140
   gaactggaag atgtatttta tcaatatgat gtaaagtctg aaatatacag ctttggaatc 1200
   gtcctctggg aaatcgccac tggagatatc ccgtttcaag gctgtaattc tgagaagatc 1260
   cgcaagctgg tggctgtgaa gcggcagcag gagccactgg gtgaagactg cccttcagag 1320
   ctgcgggaga tcattgatga gtgccgggcc catgatccct ctgtgcggcc ctctgtggat 1380
   gaaatcttaa agaaactctc caccttttct aagtag 1416

<210> 4
<211> 471
<212> PRT
<213> artificial

<220>
<223> MLKL-mutein, K230A

<400> 4

Met Glu Asn Leu Lys His Ile Ile Thr Leu Gly Gln Val Ile His Lys
1               5                   10                  15

Arg Cys Glu Glu Met Lys Tyr Cys Lys Lys Gln Cys Arg Arg Leu Gly
            20                  25                  30

His Arg Val Leu Gly Leu Ile Lys Pro Leu Glu Met Leu Gln Asp Gln
        35                  40                  45

Gly Lys Arg Ser Val Pro Ser Glu Lys Leu Thr Thr Ala Met Asn Arg
    50                  55                  60

Phe Lys Ala Ala Leu Glu Glu Ala Asn Gly Glu Ile Glu Lys Phe Ser

65                  70                  75                  80

Asn Arg Ser Asn Ile Cys Arg Phe Leu Thr Ala Ser Gln Asp Lys Ile
                85                  90                  95

Leu Phe Lys Asp Val Asn Arg Lys Leu Ser Asp Val Trp Lys Glu Leu
            100                 105                 110

Ser Leu Leu Leu Gln Val Glu Gln Arg Met Pro Val Ser Pro Ile Ser
        115                 120                 125

Gln Gly Ala Ser Trp Ala Gln Glu Asp Gln Gln Asp Ala Asp Glu Asp
    130                 135                 140

Arg Arg Ala Phe Gln Met Leu Arg Arg Asp Asn Glu Lys Ile Glu Ala
145                 150                 155                 160

Ser Leu Arg Arg Leu Glu Ile Asn Met Lys Glu Ile Lys Glu Thr Leu
                165                 170                 175

Arg Gln Tyr Leu Pro Pro Lys Cys Met Gln Glu Ile Pro Gln Glu Gln
            180                 185                 190

Ile Lys Glu Ile Lys Lys Glu Gln Leu Ser Gly Ser Pro Trp Ile Leu
        195                 200                 205

Leu Arg Glu Asn Glu Val Ser Thr Leu Tyr Lys Gly Glu Tyr His Arg
    210                 215                 220

Ala Pro Val Ala Ile Ala Val Phe Lys Lys Leu Gln Ala Gly Ser Ile
225                 230                 235                 240

Ala Ile Val Arg Gln Thr Phe Asn Lys Glu Ile Lys Thr Met Lys Lys
                245                 250                 255

Phe Glu Ser Pro Asn Ile Leu Arg Ile Phe Gly Ile Cys Ile Asp Glu
            260                 265                 270

Thr Val Thr Pro Pro Gln Phe Ser Ile Val Met Glu Tyr Cys Glu Leu
        275                 280                 285

Gly Thr Leu Arg Glu Leu Leu Asp Arg Glu Lys Asp Leu Thr Leu Gly
    290                 295                 300

Lys Arg Met Val Leu Val Leu Gly Ala Ala Arg Gly Leu Tyr Arg Leu
305                 310                 315                 320

His His Ser Glu Ala Pro Glu Leu His Gly Lys Ile Arg Ser Ser Asn
                325                 330                 335

Phe Leu Val Thr Gln Gly Tyr Gln Val Lys Leu Ala Gly Phe Glu Leu
            340                 345                 350

Arg Lys Thr Gln Thr Ser Met Ser Leu Gly Thr Thr Arg Glu Lys Thr
        355                 360                 365

Asp Arg Val Lys Ser Thr Ala Tyr Leu Ser Pro Gln Glu Leu Glu Asp
    370                 375                 380

Val Phe Tyr Gln Tyr Asp Val Lys Ser Glu Ile Tyr Ser Phe Gly Ile
385                 390                 395                 400

Val Leu Trp Glu Ile Ala Thr Gly Asp Ile Pro Phe Gln Gly Cys Asn
                405                 410                 415

Ser Glu Lys Ile Arg Lys Leu Val Ala Val Lys Arg Gln Gln Glu Pro
            420                 425                 430

Leu Gly Glu Asp Cys Pro Ser Glu Leu Arg Glu Ile Ile Asp Glu Cys
        435                 440                 445

Arg Ala His Asp Pro Ser Val Arg Pro Ser Val Asp Glu Ile Leu Lys
    450                 455                 460

Lys Leu Ser Thr Phe Ser Lys
465                 470

<210>  5
<211>  1416
<212>  DNA
<213>  artificial

<220>
<223>  MLKL-mutein, K331A

<400>  5
atggaaaatt tgaagcatat tatcaccctt ggccaggtca tccacaaacg gtgtgaagag      60

atgaaatact gcaagaaaca gtgccggcgc ctgggccacc gcgtcctcgg cctgatcaag     120

cctctggaga tgctccagga ccaaggaaag aggagcgtgc cctctgagaa gttaaccaca     180

gccatgaacc gcttcaaggc tgccctggag gaggctaatg gggagataga aaagttcagc     240

aatagatcca atatctgcag gtttctaaca gcaagccagg acaaaatact cttcaaggac     300

gtgaacagga agctgagtga tgtctggaag gagctctcgc tgttacttca ggttgagcaa     360

cgcatgcctg tttcacccat aagccaagga gcgtcctggg cacaggaaga tcagcaggat     420

gcagacgaag acaggcgagc tttccagatg ctaagaagag ataatgaaaa aatagaagct     480

tcactgagac gattagaaat caacatgaaa gaaatcaagg aaactttgag gcagtattta     540

ccaccaaaat gcatgcagga gatcccgcaa gagcaaatca aggagatcaa gaaggagcag     600

ctttcaggat ccccgtggat tctgctaagg gaaaatgaag tcagcacact ttataaagga     660

gaataccaca gagctccagt ggccataaaa gtattcaaaa aactccaggc tggcagcatt     720

gcaatagtga ggcagacttt caataaggag atcaaaacca tgaagaaatt cgaatctccc     780

aacatcctgc gtatatttgg gatttgcatt gatgaaacag tgactccgcc tcaattctcc     840

attgtcatgg agtactgtga actcgggacc ctgagggagc tgttggatag ggaaaaagac 900

ctcacacttg gcaagcgcat ggtcctagtc ctgggggcag cccgaggcct ataccggcta 960

caccattcag aagcacctga actccacgga gcaatcagaa gctcaaactt cctggtaact 1020

caaggctacc aagtgaagct tgcaggattt gagttgagga aaacacagac ttccatgagt 1080

ttgggaacta cgagagaaaa gacagacaga gtcaaatcta cagcatatct ctcacctcag 1140

gaactggaag atgtatttta tcaatatgat gtaaagtctg aaatatacag ctttggaatc 1200

gtcctctggg aaatcgccac tggagatatc ccgtttcaag gctgtaattc tgagaagatc 1260

cgcaagctgg tggctgtgaa gcggcagcag gagccactgg gtgaagactg cccttcagag 1320

ctgcgggaga tcattgatga gtgccgggcc catgatccct ctgtgcggcc ctctgtggat 1380

gaaatcttaa agaaactctc caccttttct aagtag 1416

<210> 6
<211> 471
<212> PRT
<213> artificial

<220>
<223> MLKL-mutein, K331A

<400> 6

Met Glu Asn Leu Lys His Ile Ile Thr Leu Gly Gln Val Ile His Lys
1               5                   10                  15

Arg Cys Glu Glu Met Lys Tyr Cys Lys Lys Gln Cys Arg Arg Leu Gly
            20                  25                  30

His Arg Val Leu Gly Leu Ile Lys Pro Leu Glu Met Leu Gln Asp Gln
        35                  40                  45

Gly Lys Arg Ser Val Pro Ser Glu Lys Leu Thr Thr Ala Met Asn Arg
    50                  55                  60

Phe Lys Ala Ala Leu Glu Glu Ala Asn Gly Glu Ile Glu Lys Phe Ser
65                  70                  75                  80

Asn Arg Ser Asn Ile Cys Arg Phe Leu Thr Ala Ser Gln Asp Lys Ile
                85                  90                  95

Leu Phe Lys Asp Val Asn Arg Lys Leu Ser Asp Val Trp Lys Glu Leu
            100                 105                 110

Ser Leu Leu Leu Gln Val Glu Gln Arg Met Pro Val Ser Pro Ile Ser
        115                 120                 125

Gln Gly Ala Ser Trp Ala Gln Glu Asp Gln Gln Asp Ala Asp Glu Asp
    130                 135                 140

Arg Arg Ala Phe Gln Met Leu Arg Arg Asp Asn Glu Lys Ile Glu Ala
145                 150                 155                 160

Ser Leu Arg Arg Leu Glu Ile Asn Met Lys Glu Ile Lys Glu Thr Leu
                165                 170                 175

Arg Gln Tyr Leu Pro Pro Lys Cys Met Gln Glu Ile Pro Gln Glu Gln
            180                 185                 190

Ile Lys Glu Ile Lys Lys Glu Gln Leu Ser Gly Ser Pro Trp Ile Leu
        195                 200                 205

Leu Arg Glu Asn Glu Val Ser Thr Leu Tyr Lys Gly Glu Tyr His Arg
    210                 215                 220

Ala Pro Val Ala Ile Lys Val Phe Lys Lys Leu Gln Ala Gly Ser Ile
225                 230                 235                 240

Ala Ile Val Arg Gln Thr Phe Asn Lys Glu Ile Lys Thr Met Lys Lys
                245                 250                 255

Phe Glu Ser Pro Asn Ile Leu Arg Ile Phe Gly Ile Cys Ile Asp Glu
            260                 265                 270

Thr Val Thr Pro Pro Gln Phe Ser Ile Val Met Glu Tyr Cys Glu Leu
        275                 280                 285

Gly Thr Leu Arg Glu Leu Leu Asp Arg Glu Lys Asp Leu Thr Leu Gly
    290                 295                 300

Lys Arg Met Val Leu Val Leu Gly Ala Ala Arg Gly Leu Tyr Arg Leu
305                 310                 315                 320

His His Ser Glu Ala Pro Glu Leu His Gly Ala Ile Arg Ser Ser Asn
                325                 330                 335

Phe Leu Val Thr Gln Gly Tyr Gln Val Lys Leu Ala Gly Phe Glu Leu
            340                 345                 350

Arg Lys Thr Gln Thr Ser Met Ser Leu Gly Thr Thr Arg Glu Lys Thr
        355                 360                 365

Asp Arg Val Lys Ser Thr Ala Tyr Leu Ser Pro Gln Glu Leu Glu Asp
    370                 375                 380

Val Phe Tyr Gln Tyr Asp Val Lys Ser Glu Ile Tyr Ser Phe Gly Ile
385                 390                 395                 400

Val Leu Trp Glu Ile Ala Thr Gly Asp Ile Pro Phe Gln Gly Cys Asn
                405                 410                 415

Ser Glu Lys Ile Arg Lys Leu Val Ala Val Lys Arg Gln Gln Glu Pro
            420                 425                 430

Leu Gly Glu Asp Cys Pro Ser Glu Leu Arg Glu Ile Ile Asp Glu Cys

435                 440                 445

Arg Ala His Asp Pro Ser Val Arg Pro Ser Val Asp Glu Ile Leu Lys
    450                 455                 460

Lys Leu Ser Thr Phe Ser Lys
465                 470

<210>  7
<211>  1395
<212>  DNA
<213>  Mus musculus

<400>  7
atggataaat tgggacagat catcaagtta ggccagctca tctatgaaca gtgtgaaaag      60

atgaaatact gccggaaaca atgccagcgt ctaggaaacc gtgtgcacgg cctgctacag     120

cctctccaga gactccaggc ccaaggaaag aagaacctgc ccgatgacat tactgctgcc     180

ctgggccgtt ttgatgaagt cctgaaggag gctaaccagc agatagaaaa gttcagcaag     240

aagtcccata tttggaagtt tgtgagtgtg ggcaatgata agatcctctt ccatgaagtg     300

aatgagaagc tgagagacgt ctgggaggag ctgttgctgc tgcttcaggt ttatcattgg     360

aataccgttt cagatgtcag ccagccagca tcctggcagc aggaagatcg acaggatgca     420

gaggaagacg gaaatgaaaa tatgaaagtt atcctgatgc agttgcaaat tagcgtggaa     480

gaaatcaaca aaaccctgaa gcaatgctca ctaaaaccca cacaggagat cccacaagat     540

ctccaaatca aggagattcc aaaggaacat cttggacctc cgtggaccaa actgaagaca     600

agtaaaatga gcaccattta tagaggagag tatcacagat ctccagttac catcaaagta     660

ttcaacaacc cccaggccga aagtgttgga atagtgaggt tcactttcaa tgacgagatc     720

aaaaccatga agaaattcga ttctcccaac atcttgcgta tatttgggat ttgcattgat     780

caaacagtga agccccctga gttctccatt gtcatggagt actgtgaact tggaaccctg     840

agggaactgc tggatagaga aaaagacctc acaatgagtg tgcgcagcct cctagtcctg     900

agggcagcca gaggcttata caggctacac cattcggaaa cactccacag aaacatcagc     960

agctccagtt tcctcgtagc cggaggctac caagtaaagc ttgcaggatt tgagttaagc    1020

aaaacacaga attccatcag ccggacagca aagagcacta aagcagagag atccagttca    1080

acgatatatg tctcccctga gagactgaaa aatccatttt gcctttatga cataaaagct    1140

gaaatatata gctttggaat tgtactctgg gaaattgcca ctggaaagat cccatttgaa    1200

ggctgtgatt ctaagaagat ccgggagctg gtggctgagg acaagaagca ggaaccagtg    1260

ggtcaggatt gccctgagtt gttgcgggaa atcattaatg agtgtcgtgc ccatgagccc    1320

tcccaacggc cctctgtgga cggaatcttg gagagactgt ctgcggttga agaatccacg    1380

gacaagaagg tgtaa                                                     1395

<210>  8
<211>  464
<212>  PRT
<213>  Mus musculus

<400>  8

Met Asp Lys Leu Gly Gln Ile Ile Lys Leu Gly Gln Leu Ile Tyr Glu
1               5                   10                  15

Gln Cys Glu Lys Met Lys Tyr Cys Arg Lys Gln Cys Gln Arg Leu Gly
            20                  25                  30

Asn Arg Val His Gly Leu Leu Gln Pro Leu Gln Arg Leu Gln Ala Gln
        35                  40                  45

Gly Lys Lys Asn Leu Pro Asp Asp Ile Thr Ala Ala Leu Gly Arg Phe
    50                  55                  60

Asp Glu Val Leu Lys Glu Ala Asn Gln Gln Ile Glu Lys Phe Ser Lys
65                  70                  75                  80

Lys Ser His Ile Trp Lys Phe Val Ser Val Gly Asn Asp Lys Ile Leu
                85                  90                  95

Phe His Glu Val Asn Glu Lys Leu Arg Asp Val Trp Glu Glu Leu Leu
            100                 105                 110

Leu Leu Leu Gln Val Tyr His Trp Asn Thr Val Ser Asp Val Ser Gln
        115                 120                 125

Pro Ala Ser Trp Gln Gln Glu Asp Arg Gln Asp Ala Glu Glu Asp Gly
    130                 135                 140

Asn Glu Asn Met Lys Val Ile Leu Met Gln Leu Gln Ile Ser Val Glu
145                 150                 155                 160

Glu Ile Asn Lys Thr Leu Lys Gln Cys Ser Leu Lys Pro Thr Gln Glu
                165                 170                 175

Ile Pro Gln Asp Leu Gln Ile Lys Glu Ile Pro Lys Glu His Leu Gly
            180                 185                 190

Pro Pro Trp Thr Lys Leu Lys Thr Ser Lys Met Ser Thr Ile Tyr Arg
        195                 200                 205

Gly Glu Tyr His Arg Ser Pro Val Thr Ile Lys Val Phe Asn Asn Pro
    210                 215                 220

Gln Ala Glu Ser Val Gly Ile Val Arg Phe Thr Phe Asn Asp Glu Ile
225                 230                 235                 240

Lys Thr Met Lys Lys Phe Asp Ser Pro Asn Ile Leu Arg Ile Phe Gly
                245                 250                 255

Ile Cys Ile Asp Gln Thr Val Lys Pro Pro Glu Phe Ser Ile Val Met
            260                 265                 270

Glu Tyr Cys Glu Leu Gly Thr Leu Arg Glu Leu Leu Asp Arg Glu Lys
        275                 280                 285

Asp Leu Thr Met Ser Val Arg Ser Leu Leu Val Leu Arg Ala Ala Arg
    290                 295                 300

Gly Leu Tyr Arg Leu His His Ser Glu Thr Leu His Arg Asn Ile Ser
305                 310                 315                 320

Ser Ser Ser Phe Leu Val Ala Gly Gly Tyr Gln Val Lys Leu Ala Gly
                325                 330                 335

Phe Glu Leu Ser Lys Thr Gln Asn Ser Ile Ser Arg Thr Ala Lys Ser
            340                 345                 350

Thr Lys Ala Glu Arg Ser Ser Ser Thr Ile Tyr Val Ser Pro Glu Arg
        355                 360                 365

Leu Lys Asn Pro Phe Cys Leu Tyr Asp Ile Lys Ala Glu Ile Tyr Ser
    370                 375                 380

Phe Gly Ile Val Leu Trp Glu Ile Ala Thr Gly Lys Ile Pro Phe Glu
385                 390                 395                 400

Gly Cys Asp Ser Lys Lys Ile Arg Glu Leu Val Ala Glu Asp Lys Lys
                405                 410                 415

Gln Glu Pro Val Gly Gln Asp Cys Pro Glu Leu Leu Arg Glu Ile Ile
            420                 425                 430

Asn Glu Cys Arg Ala His Glu Pro Ser Gln Arg Pro Ser Val Asp Gly
        435                 440                 445

Ile Leu Glu Arg Leu Ser Ala Val Glu Glu Ser Thr Asp Lys Lys Val
    450                 455                 460

<210>  9
<211>  1386
<212>  DNA
<213>  Rattus norvegicus

<400>  9
atggataaat tgggacagat catcaagtta ggccagctca tctatgacca gtgtgaaaag      60

atgaaatact gcaaaagaca gtgccggcgt ctaagaaacc gtgtgcatgg cctgctacag     120

cctctccaga ggctccaggc ccaagaaaag aagagcctcc ccagtgacat tactgctgcc     180

ctgggccgtt ttgaggacgt cctaaaagaa gctagacagc agatagaaaa gttcagtcag     240

aagtccaata tttggaagtt tgctactgtg ggcagtgata agatcctctt ccatgaagtg     300

aatgagaagc tgagagacat ctgggaggag ctcttgctgc tgcttcaggt tgatcaatgg     360

aagtctgatt ccgaggtcag ccaaccagca tcctggcagc aggaagatca acaggacgca     420

gaggaagatg gaaatgaaaa tatgaaagtt attttggtga agctgcaaat cagcatggaa     480

gaaatcaaag aggccctgaa gcaatgctca ctaaaaccca tgcaggaggt cccacaagat 540

ttccaagtca aggagattcc gaaggaagat cttggatatc cgtggaccaa actgaagaca 600

aataaactga gcacgattta tagaggagag tattacaaat ctccagtcac catcaaagta 660

ttcaacaacc ccaaggccga aagtgttgga atagtgaggc tcactttcaa tgaggagatc 720

aaaaccatga agaaatttga ttctcccaac atcctgcgta tatttgggat ttgcatcgat 780

caaacagtga agccccccga attctccatt gtcatggagt actgtgaact cggaaccctg 840

agggaactgc tggatagaga caaggacctc accatgagtg tgcgcagcct cctagtcctg 900

agggcagcta gaggcttgta caggttacac cactcgggaa cactccacgg aaacatcagc 960

agctccagct tcctggtagc tggaggctac caagtaaagc ttgcaggatt tgagctaagc 1020

aaaacacaga cttctataag ccgggcaaca aagagcacta aagcagagag attcagttcg 1080

acagcatatg tctcccccga gagactgcaa gatccatttt gcaaatatga cacaaaagct 1140

gaaatatata gctttggaat tgtgctctgg gaaattgcca ctggaaagat cccgtttgaa 1200

ggctgtgatt ctgagaagat ctatgagctg gtggctgaga acaagaagca ggagccagtg 1260

ggtcaggatt gccccgagtt gttgcaggag atcattaatg agtgccgggc ccatgaaccc 1320

tccaatcggc cctctgcgga tggtaggatt ctttctgtca aagagtgcaa gctgtgtgtt 1380

ttgtga 1386

<210> 10
<211> 461
<212> PRT
<213> Rattus norvegicus

<400> 10

Met Asp Lys Leu Gly Gln Ile Ile Lys Leu Gly Gln Leu Ile Tyr Asp
1               5                   10                  15

Gln Cys Glu Lys Met Lys Tyr Cys Lys Arg Gln Cys Arg Arg Leu Arg
            20                  25                  30

Asn Arg Val His Gly Leu Leu Gln Pro Leu Gln Arg Leu Gln Ala Gln
        35                  40                  45

Glu Lys Lys Ser Leu Pro Ser Asp Ile Thr Ala Ala Leu Gly Arg Phe
    50                  55                  60

Glu Asp Val Leu Lys Glu Ala Arg Gln Gln Ile Glu Lys Phe Ser Gln
65                  70                  75                  80

Lys Ser Asn Ile Trp Lys Phe Ala Thr Val Gly Ser Asp Lys Ile Leu
                85                  90                  95

Phe His Glu Val Asn Glu Lys Leu Arg Asp Ile Trp Glu Glu Leu Leu
            100                 105                 110

Leu Leu Leu Gln Val Asp Gln Trp Lys Ser Asp Ser Glu Val Ser Gln

115                 120                 125

Pro Ala Ser Trp Gln Gln Glu Asp Gln Gln Asp Ala Glu Glu Asp Gly
    130                 135                 140

Asn Glu Asn Met Lys Val Ile Leu Val Lys Leu Gln Ile Ser Met Glu
145                 150                 155                 160

Glu Ile Lys Glu Ala Leu Lys Gln Cys Ser Leu Lys Pro Met Gln Glu
                165                 170                 175

Val Pro Gln Asp Phe Gln Val Lys Glu Ile Pro Lys Glu Asp Leu Gly
            180                 185                 190

Tyr Pro Trp Thr Lys Leu Lys Thr Asn Lys Leu Ser Thr Ile Tyr Arg
        195                 200                 205

Gly Glu Tyr Tyr Lys Ser Pro Val Thr Ile Lys Val Phe Asn Asn Pro
    210                 215                 220

Lys Ala Glu Ser Val Gly Ile Val Arg Leu Thr Phe Asn Glu Glu Ile
225                 230                 235                 240

Lys Thr Met Lys Lys Phe Asp Ser Pro Asn Ile Leu Arg Ile Phe Gly
                245                 250                 255

Ile Cys Ile Asp Gln Thr Val Lys Pro Pro Glu Phe Ser Ile Val Met
            260                 265                 270

Glu Tyr Cys Glu Leu Gly Thr Leu Arg Glu Leu Leu Asp Arg Asp Lys
        275                 280                 285

Asp Leu Thr Met Ser Val Arg Ser Leu Leu Val Leu Arg Ala Ala Arg
    290                 295                 300

Gly Leu Tyr Arg Leu His His Ser Gly Thr Leu His Gly Asn Ile Ser
305                 310                 315                 320

Ser Ser Ser Phe Leu Val Ala Gly Gly Tyr Gln Val Lys Leu Ala Gly
                325                 330                 335

Phe Glu Leu Ser Lys Thr Gln Thr Ser Ile Ser Arg Ala Thr Lys Ser
            340                 345                 350

Thr Lys Ala Glu Arg Phe Ser Ser Thr Ala Tyr Val Ser Pro Glu Arg
        355                 360                 365

Leu Gln Asp Pro Phe Cys Lys Tyr Asp Thr Lys Ala Glu Ile Tyr Ser
    370                 375                 380

Phe Gly Ile Val Leu Trp Glu Ile Ala Thr Gly Lys Ile Pro Phe Glu
385                 390                 395                 400

Gly Cys Asp Ser Glu Lys Ile Tyr Glu Leu Val Ala Glu Asn Lys Lys
                405                 410                 415

Gln Glu Pro Val Gly Gln Asp Cys Pro Glu Leu Leu Gln Glu Ile Ile
            420                 425                 430

Asn Glu Cys Arg Ala His Glu Pro Ser Asn Arg Pro Ser Ala Asp Gly
        435                 440                 445

Arg Ile Leu Ser Val Lys Glu Cys Lys Leu Cys Val Leu
    450                 455                 460

<210>  11
<211>  1416
<212>  DNA
<213>  Pan troglodytes

<400>  11
atggaaaatt tgaagcatat tatcaccctt ggccaggtca tccacaaacg gtgtgaagag      60

atgaaatact gcaagaaaca gtgccggcgc ctgggccacc gcgtcctcgg cctgatcaag     120

cctctggaga tgctccagga ccaaggaaag aggagcgtgc cctctgagaa gttaaccaca     180

gccatgaacc gcttcaaggc tgccctggag gaggctaatg gggagataga aaagttcagc     240

aatagatcca atatctgcag gtttctaaca gcaagccagg acaaaatact cttcaaggac     300

gtgaacagga agctgagtga tgtctggaag gagctctcgc tgttacttca ggttgagcaa     360

cgcatgcctg tttcacccat aagccaagga gcgtcctggg cacaggaaga tcagcaggat     420

gcagacgaag acaggcgagc cttccagatg ctaagaagag ataatgaaaa aatagaagct     480

tcattgagac gattagaaat caacatgaaa gaaatcaagg aaactttgag gcagtattta     540

ccaccaaaat gcatgcagga gatcccgcaa gagcaaatca aggagatcaa gaaggagcag     600

ctttcaggat ccccgtggat tctgctaagg gaaaatgaag tcagcacact ttataaagga     660

gaataccaca gagctccagt ggccataaaa gtattcaaaa aactccaggc tggcagcatt     720

gcaatagtga ggcagacttt caataatgag atcaaaacca tgaagaaatt cgaatctccc     780

aacatcctgc gtatatttgg gatttgcatt gatgaaacag tgactccgcc tcaattctcc     840

gttgtcatgg agtactgtga actcgggacc ctgagggagc tgttggatag ggaaaaagac     900

ctcacacttg gcaagcgcat ggtcctagtc ctgggggcag cccgaggcct gtaccggcta     960

caccattcag aagcacctga actccacgga aaaatcagaa gctcaaactt cctggtaact    1020

caaggctacc aagtgaagct tgcaggattt gagttgaggg aaacacagac ttccatgagt    1080

ttgggaacta cgagagaaaa gacagacaaa gtcaaatcta cagcatatct ctcacctcag    1140

gaactggaag acgtatttta tctatatgat gtaaagtctg aaatatacag ctttggaatc    1200

gtcctctggg aaatcgccac tggagatatc ccgtttcaag gctgtaattc tgaggagatc    1260

cgcaagctgg tgtctgtgga gcgggagcag gagccgctgg gtgaagactg cccttcagag    1320

ctgcgggaga tcattgatca gtgccgggcc catgatccct ctttgcggcc ctccgtggat    1380

gaaatcttaa agaaactctc cacctttctt aagtag                              1416

<210>  12
<211>  471
<212>  PRT
<213>  Pan troglodytes

<400>  12

Met Glu Asn Leu Lys His Ile Ile Thr Leu Gly Gln Val Ile His Lys
1               5                   10                  15

Arg Cys Glu Glu Met Lys Tyr Cys Lys Lys Gln Cys Arg Arg Leu Gly
            20                  25                  30

His Arg Val Leu Gly Leu Ile Lys Pro Leu Glu Met Leu Gln Asp Gln
        35                  40                  45

Gly Lys Arg Ser Val Pro Ser Glu Lys Leu Thr Thr Ala Met Asn Arg
    50                  55                  60

Phe Lys Ala Ala Leu Glu Glu Ala Asn Gly Glu Ile Glu Lys Phe Ser
65                  70                  75                  80

Asn Arg Ser Asn Ile Cys Arg Phe Leu Thr Ala Ser Gln Asp Lys Ile
                85                  90                  95

Leu Phe Lys Asp Val Asn Arg Lys Leu Ser Asp Val Trp Lys Glu Leu
            100                 105                 110

Ser Leu Leu Leu Gln Val Glu Gln Arg Met Pro Val Ser Pro Ile Ser
        115                 120                 125

Gln Gly Ala Ser Trp Ala Gln Glu Asp Gln Gln Asp Ala Asp Glu Asp
    130                 135                 140

Arg Arg Ala Phe Gln Met Leu Arg Arg Asp Asn Glu Lys Ile Glu Ala
145                 150                 155                 160

Ser Leu Arg Arg Leu Glu Ile Asn Met Lys Glu Ile Lys Glu Thr Leu
                165                 170                 175

Arg Gln Tyr Leu Pro Pro Lys Cys Met Gln Glu Ile Pro Gln Glu Gln
            180                 185                 190

Ile Lys Glu Ile Lys Lys Glu Gln Leu Ser Gly Ser Pro Trp Ile Leu
        195                 200                 205

Leu Arg Glu Asn Glu Val Ser Thr Leu Tyr Lys Gly Glu Tyr His Arg
    210                 215                 220

Ala Pro Val Ala Ile Lys Val Phe Lys Lys Leu Gln Ala Gly Ser Ile
225                 230                 235                 240

Ala Ile Val Arg Gln Thr Phe Asn Asn Glu Ile Lys Thr Met Lys Lys

245                 250                 255

Phe Glu Ser Pro Asn Ile Leu Arg Ile Phe Gly Ile Cys Ile Asp Glu
            260                 265                 270

Thr Val Thr Pro Pro Gln Phe Ser Val Val Met Glu Tyr Cys Glu Leu
        275                 280                 285

Gly Thr Leu Arg Glu Leu Leu Asp Arg Glu Lys Asp Leu Thr Leu Gly
    290                 295                 300

Lys Arg Met Val Leu Val Leu Gly Ala Ala Arg Gly Leu Tyr Arg Leu
305                 310                 315                 320

His His Ser Glu Ala Pro Glu Leu His Gly Lys Ile Arg Ser Ser Asn
                325                 330                 335

Phe Leu Val Thr Gln Gly Tyr Gln Val Lys Leu Ala Gly Phe Glu Leu
            340                 345                 350

Arg Glu Thr Gln Thr Ser Met Ser Leu Gly Thr Thr Arg Glu Lys Thr
        355                 360                 365

Asp Lys Val Lys Ser Thr Ala Tyr Leu Ser Pro Gln Glu Leu Glu Asp
    370                 375                 380

Val Phe Tyr Leu Tyr Asp Val Lys Ser Glu Ile Tyr Ser Phe Gly Ile
385                 390                 395                 400

Val Leu Trp Glu Ile Ala Thr Gly Asp Ile Pro Phe Gln Gly Cys Asn
                405                 410                 415

Ser Glu Glu Ile Arg Lys Leu Val Ser Val Glu Arg Glu Gln Glu Pro
            420                 425                 430

Leu Gly Glu Asp Cys Pro Ser Glu Leu Arg Glu Ile Ile Asp Gln Cys
        435                 440                 445

Arg Ala His Asp Pro Ser Leu Arg Pro Ser Val Asp Glu Ile Leu Lys
    450                 455                 460

Lys Leu Ser Thr Phe Ser Lys
465                 470

<210>  13
<211>  792
<212>  DNA
<213>  Pan troglodytes

<400>  13
atggaaaatt tgaagcatat tatcaccctt ggccaggtca tccacaaacg gtgtgaagag      60

atgaaatact gcaagaaaca gtgccggcgc ctgggccacc gcgtcctcgg cctgatcaag     120

cctctggaga tgctccagga ccaaggaaag aggagcgtgc cctctgagaa gttaaccaca     180

gccatgaacc gcttcaaggc tgccctggag gaggctaatg gggagataga aaagttcagc 240

aatagatcca atatctgcag gtttctaaca gcaagccagg acaaaatact cttcaaggac 300

gtgaacagga agctgagtga tgtctggaag gagctctcgc tgttacttca ggttgagcaa 360

cgcatgcctg tttcacccat aagccaagga gcgtcctggg cacaggaaga tcagcaggat 420

gcagacgaag acaggcgagc cttccagatg ctaagaagag ataatgaaaa aatagaagct 480

tcattgagac gattagaaat caacatgaaa gaaatcaagg aaactttgag gcagtctttg 540

gaatcgtcct ctgggaaatc gccactggag atatcccgtt tcaaggtgaa gaatgtgaag 600

actggctcag ccagtggctg taattctgag gagatccgca agctggtgtc tgtggagcgg 660

gagcaggagc cgctgggtga agactgccct tcagagctgc gggagatcat tgatcagtgc 720

cgggcccatg atccctcttt gcggccctcc gtggatgaaa tcttaaagaa actctccacc 780

ttttctaagt ag 792

<210> 14
<211> 263
<212> PRT
<213> Pan troglodytes

<400> 14

Met Glu Asn Leu Lys His Ile Ile Thr Leu Gly Gln Val Ile His Lys
1               5                   10                  15

Arg Cys Glu Glu Met Lys Tyr Cys Lys Lys Gln Cys Arg Arg Leu Gly
            20                  25                  30

His Arg Val Leu Gly Leu Ile Lys Pro Leu Glu Met Leu Gln Asp Gln
        35                  40                  45

Gly Lys Arg Ser Val Pro Ser Glu Lys Leu Thr Thr Ala Met Asn Arg
    50                  55                  60

Phe Lys Ala Ala Leu Glu Glu Ala Asn Gly Glu Ile Glu Lys Phe Ser
65                  70                  75                  80

Asn Arg Ser Asn Ile Cys Arg Phe Leu Thr Ala Ser Gln Asp Lys Ile
                85                  90                  95

Leu Phe Lys Asp Val Asn Arg Lys Leu Ser Asp Val Trp Lys Glu Leu
            100                 105                 110

Ser Leu Leu Leu Gln Val Glu Gln Arg Met Pro Val Ser Pro Ile Ser
        115                 120                 125

Gln Gly Ala Ser Trp Ala Gln Glu Asp Gln Gln Asp Ala Asp Glu Asp
    130                 135                 140

Arg Arg Ala Phe Gln Met Leu Arg Arg Asp Asn Glu Lys Ile Glu Ala
145                 150                 155                 160

Ser Leu Arg Arg Leu Glu Ile Asn Met Lys Glu Ile Lys Glu Thr Leu
                165                 170                 175

Arg Gln Ser Leu Glu Ser Ser Ser Gly Lys Ser Pro Leu Glu Ile Ser
            180                 185                 190

Arg Phe Lys Val Lys Asn Val Lys Thr Gly Ser Ala Ser Gly Cys Asn
        195                 200                 205

Ser Glu Glu Ile Arg Lys Leu Val Ser Val Glu Arg Glu Gln Glu Pro
    210                 215                 220

Leu Gly Glu Asp Cys Pro Ser Glu Leu Arg Glu Ile Ile Asp Gln Cys
225                 230                 235                 240

Arg Ala His Asp Pro Ser Leu Arg Pro Ser Val Asp Glu Ile Leu Lys
                245                 250                 255

Lys Leu Ser Thr Phe Ser Lys
            260

<210>  15
<211>  825
<212>  DNA
<213>  artificial

<220>
<223>  ANKRD7

<400>  15
atgaataagc ttttcagctt ctggaagagg aagaatgaga cccgcagcca gggctacaac      60

cttcgagaaa aggatttaaa gaaacttcac agagctgctt cagtcgggga tttgaagaag     120

ctgaaggaat accttcagat caagaaatat gatgtaaata tgcaggacaa aaaatacagt     180

gttgtccaat cttctagatg gcttttatcc tacaccactc cgctgaatga ctttataaga     240

acacctttgc acctagcctg tgctaatgga catacagatg ttgtactttt cctaattgag     300

caacaatgca aaataaatgt ccgggatagt gaaaacaaat ccccattgat taaggcagta     360

cagtgtcaaa atgaggattg tgctactatt cttctaaact ttggtgcaga cccagatctg     420

agggatattc gttataatac tgttcttcac tatgctgttt gtggtcaaag tttgtcatta     480

gttgaaaaac tgcttgaata cgaagctgat cttgaagcga aaaataagga tgggtatact     540

ccactattag ttgccgttat taacaataat ccaaaaatgg taaaatttct tctggagaaa     600

ggggctgatg tgaatgcttc agataattat caaagaacag cccttattct tgctgtcagt     660

ggtgaaccac catgtttagt aaagcttctt cttcagcaag gtgtggaatt atgttacgaa     720

ggtattgtgg attcacagct gaggaatatg tttatttcca tggttttact gcatagatac     780

ccacaattca ctgcgagcca tggaaagaag aaacatgcta aatag                     825

<210>  16
<211>  274
<212>  PRT

<213>  artificial

<220>
<223>  ANKRD7

<400>  16

Met Asn Lys Leu Phe Ser Phe Trp Lys Arg Lys Asn Glu Thr Arg Ser
1               5                   10                  15

Gln Gly Tyr Asn Leu Arg Glu Lys Asp Leu Lys Lys Leu His Arg Ala
            20                  25                  30

Ala Ser Val Gly Asp Leu Lys Lys Leu Lys Glu Tyr Leu Gln Ile Lys
        35                  40                  45

Lys Tyr Asp Val Asn Met Gln Asp Lys Lys Tyr Ser Val Val Gln Ser
    50                  55                  60

Ser Arg Trp Leu Leu Ser Tyr Thr Thr Pro Leu Asn Asp Phe Ile Arg
65                  70                  75                  80

Thr Pro Leu His Leu Ala Cys Ala Asn Gly His Thr Asp Val Val Leu
                85                  90                  95

Phe Leu Ile Glu Gln Gln Cys Lys Ile Asn Val Arg Asp Ser Glu Asn
            100                 105                 110

Lys Ser Pro Leu Ile Lys Ala Val Gln Cys Gln Asn Glu Asp Cys Ala
        115                 120                 125

Thr Ile Leu Leu Asn Phe Gly Ala Asp Pro Asp Leu Arg Asp Ile Arg
    130                 135                 140

Tyr Asn Thr Val Leu His Tyr Ala Val Cys Gly Gln Ser Leu Ser Leu
145                 150                 155                 160

Val Glu Lys Leu Leu Glu Tyr Glu Ala Asp Leu Glu Ala Lys Asn Lys
                165                 170                 175

Asp Gly Tyr Thr Pro Leu Leu Val Ala Val Ile Asn Asn Asn Pro Lys
            180                 185                 190

Met Val Lys Phe Leu Leu Glu Lys Gly Ala Asp Val Asn Ala Ser Asp
        195                 200                 205

Asn Tyr Gln Arg Thr Ala Leu Ile Leu Ala Val Ser Gly Glu Pro Pro
    210                 215                 220

Cys Leu Val Lys Leu Leu Leu Gln Gln Gly Val Glu Leu Cys Tyr Glu
225                 230                 235                 240

Gly Ile Val Asp Ser Gln Leu Arg Asn Met Phe Ile Ser Met Val Leu
                245                 250                 255

Leu His Arg Tyr Pro Gln Phe Thr Ala Ser His Gly Lys Lys Lys His
            260                 265                 270

Ala Lys

<210>  17
<211>  765
<212>  DNA
<213>  Homo sapiens

<400>  17
atgaataagc ttttcagctt ctggaagagg aagaatgaga cccgcagcca gggctacaac      60

cttcgagaaa aggatttaaa gaaacttcac agagctgctt cagtcgggga tttgaagaag     120

ctgaaggaat accttcagat caagaaatat gatgtaaata tgcaggacaa aaaatacaga     180

acacctttgc acctagcctg tgctaatgga catacagatg ttgtactttt cctaattgag     240

caacaatgca aaataaatgt ccgggatagt gaaaacaaat ccccattgat taaggcagta     300

cagtgtcaaa atgaggattg tgctactatt cttctaaact ttggtgcaga cccagatctg     360

agggatattc gttataatac tgttcttcac tatgctgttt gtggtcaaag tttgtcatta     420

gttgaaaaac tgcttgaata cgaagctgat cttgaagcga aaaataagga tgggtatact     480

ccactattag ttgccgttat taacaataat ccaaaaatgg taaaatttct tctggagaaa     540

ggggctgatg tgaatgcttc agataattat caaagaacag cccttattct tgctgtcagt     600

ggtgaaccac catgtttagt aaagcttctt cttcagcaag gtgtggaatt atgttacgaa     660

ggtattgtgg attcacagct gaggaatatg tttatttcca tggttttact gcatagatac     720

ccacaattca ctgcgagcca tggaaagaag aaacatgcta aatag                     765

<210>  18
<211>  254
<212>  PRT
<213>  Homo sapiens

<400>  18

Met Asn Lys Leu Phe Ser Phe Trp Lys Arg Lys Asn Glu Thr Arg Ser
1               5                   10                  15

Gln Gly Tyr Asn Leu Arg Glu Lys Asp Leu Lys Lys Leu His Arg Ala
            20                  25                  30

Ala Ser Val Gly Asp Leu Lys Lys Leu Lys Glu Tyr Leu Gln Ile Lys
        35                  40                  45

Lys Tyr Asp Val Asn Met Gln Asp Lys Lys Tyr Arg Thr Pro Leu His
    50                  55                  60

Leu Ala Cys Ala Asn Gly His Thr Asp Val Val Leu Phe Leu Ile Glu
65                  70                  75                  80

Gln Gln Cys Lys Ile Asn Val Arg Asp Ser Glu Asn Lys Ser Pro Leu

                85                  90                  95

Ile Lys Ala Val Gln Cys Gln Asn Glu Asp Cys Ala Thr Ile Leu Leu
            100                 105                 110

Asn Phe Gly Ala Asp Pro Asp Leu Arg Asp Ile Arg Tyr Asn Thr Val
        115                 120                 125

Leu His Tyr Ala Val Cys Gly Gln Ser Leu Ser Leu Val Glu Lys Leu
    130                 135                 140

Leu Glu Tyr Glu Ala Asp Leu Glu Ala Lys Asn Lys Asp Gly Tyr Thr
145                 150                 155                 160

Pro Leu Leu Val Ala Val Ile Asn Asn Asn Pro Lys Met Val Lys Phe
                165                 170                 175

Leu Leu Glu Lys Gly Ala Asp Val Asn Ala Ser Asp Asn Tyr Gln Arg
            180                 185                 190

Thr Ala Leu Ile Leu Ala Val Ser Gly Glu Pro Pro Cys Leu Val Lys
        195                 200                 205

Leu Leu Leu Gln Gln Gly Val Glu Leu Cys Tyr Glu Gly Ile Val Asp
    210                 215                 220

Ser Gln Leu Arg Asn Met Phe Ile Ser Met Val Leu Leu His Arg Tyr
225                 230                 235                 240

Pro Gln Phe Thr Ala Ser His Gly Lys Lys Lys His Ala Lys
                245                 250

<210> 19
<211> 1557
<212> DNA
<213> artificial

<220>
<223> RIPK3-mRNA-sequence (coding sequence)

<400> 19
atgtcgtgcg tcaagttatg gcccagcggt gcccccgccc ccttggtgtc catcgaggaa 60

ctggagaacc aggagctcgt cggcaaaggc gggttcggca cagtgttccg ggcgcaacat 120

aggaagtggg gctacgatgt ggcggtcaag atcgtaaact cgaaggcgat atccagggag 180

gtcaaggcca tggcaagtct ggataacgaa ttcgtgctgc gcctagaagg ggttatcgag 240

aaggtgaact gggaccaaga tcccaagccg gctctggtga ctaaattcat ggagaacggc 300

tccttgtcgg ggctgctgca gtcccagtgc cctcggccct ggccgctcct ttgccgcctg 360

ctgaaagaag tggtgcttgg gatgttttac ctgcacgacc agaacccggt gctcctgcac 420

cgggacctca agccatccaa cgtcctgctg gacccagagc tgcacgtcaa gctggcagat 480

tttggcctgt ccacatttca gggaggctca cagtcaggga cagggtccgg ggagccaggg 540

ggcaccctgg gctacttggc cccagaactg tttgttaacg taaaccggaa ggcctccaca      600

gccagtgacg tctacagctt cgggatccta atgtgggcag tgcttgctgg aagagaagtt      660

gagttgccaa ccgaaccatc actcgtgtac gaagcagtgt gcaacaggca gaaccggcct      720

tcattggctg agctgcccca agccgggcct gagactcccg gcttagaagg actgaaggag      780

ctaatgcagc tctgctggag cagtgagccc aaggacagac cctccttcca ggaatgccta      840

ccaaaaactg atgaagtctt ccagatggtg gagaacaata tgaatgctgc tgtctccacg      900

gtaaaggatt tcctgtctca gctcaggagc agcaatagga gattttctat cccagagtca      960

ggccaaggag ggacagaaat ggatggcttt aggagaacca tagaaaacca gcactctcgt     1020

aatgatgtca tggtttctga gtggctaaac aaactgaatc tagaggagcc tcccagctct     1080

gttcctaaaa aatgcccgag ccttaccaag aggagcaggg cacaagagga gcaggttcca     1140

caagcctgga cagcaggcac atcttcagat tcgatggccc aacctcccca gactccagag     1200

acctcaactt tcagaaacca gatgcccagc cctacctcaa ctggaacacc aagtcctgga     1260

ccccgaggga atcagggggc tgagagacaa ggcatgaact ggtcctgcag gaccccggag      1320

ccaaatccag taacagggcg accgctcgtt aacatataca actgctctgg ggtgcaagtt     1380

ggagacaaca actacttgac tatgcaacag acaactgcct tgcccacatg gggcttggca      1440

ccttcgggca aggggagggg cttgcagcac cccccaccag taggttcgca agaaggccct     1500

aaagatcctg aagcctggag caggccacag ggttggtata atcatagcgg gaaataa        1557

<210>  20
<211>  518
<212>  PRT
<213>  artificial

<220>
<223>  RIPK3 amino acid sequence (coding sequence)

<400>  20

Met Ser Cys Val Lys Leu Trp Pro Ser Gly Ala Pro Ala Pro Leu Val
1               5                   10                  15

Ser Ile Glu Glu Leu Glu Asn Gln Glu Leu Val Gly Lys Gly Gly Phe
            20                  25                  30

Gly Thr Val Phe Arg Ala Gln His Arg Lys Trp Gly Tyr Asp Val Ala
        35                  40                  45

Val Lys Ile Val Asn Ser Lys Ala Ile Ser Arg Glu Val Lys Ala Met
    50                  55                  60

Ala Ser Leu Asp Asn Glu Phe Val Leu Arg Leu Glu Gly Val Ile Glu
65                  70                  75                  80

Lys Val Asn Trp Asp Gln Asp Pro Lys Pro Ala Leu Val Thr Lys Phe
                85                  90                  95

Met Glu Asn Gly Ser Leu Ser Gly Leu Leu Gln Ser Gln Cys Pro Arg

            100                 105                 110

Pro Trp Pro Leu Leu Cys Arg Leu Leu Lys Glu Val Val Leu Gly Met
        115                 120                 125

Phe Tyr Leu His Asp Gln Asn Pro Val Leu Leu His Arg Asp Leu Lys
    130                 135                 140

Pro Ser Asn Val Leu Leu Asp Pro Glu Leu His Val Lys Leu Ala Asp
145                 150                 155                 160

Phe Gly Leu Ser Thr Phe Gln Gly Gly Ser Gln Ser Gly Thr Gly Ser
                165                 170                 175

Gly Glu Pro Gly Gly Thr Leu Gly Tyr Leu Ala Pro Glu Leu Phe Val
            180                 185                 190

Asn Val Asn Arg Lys Ala Ser Thr Ala Ser Asp Val Tyr Ser Phe Gly
        195                 200                 205

Ile Leu Met Trp Ala Val Leu Ala Gly Arg Glu Val Glu Leu Pro Thr
    210                 215                 220

Glu Pro Ser Leu Val Tyr Glu Ala Val Cys Asn Arg Gln Asn Arg Pro
225                 230                 235                 240

Ser Leu Ala Glu Leu Pro Gln Ala Gly Pro Glu Thr Pro Gly Leu Glu
                245                 250                 255

Gly Leu Lys Glu Leu Met Gln Leu Cys Trp Ser Ser Glu Pro Lys Asp
            260                 265                 270

Arg Pro Ser Phe Gln Glu Cys Leu Pro Lys Thr Asp Glu Val Phe Gln
        275                 280                 285

Met Val Glu Asn Asn Met Asn Ala Ala Val Ser Thr Val Lys Asp Phe
    290                 295                 300

Leu Ser Gln Leu Arg Ser Ser Asn Arg Arg Phe Ser Ile Pro Glu Ser
305                 310                 315                 320

Gly Gln Gly Gly Thr Glu Met Asp Gly Phe Arg Arg Thr Ile Glu Asn
                325                 330                 335

Gln His Ser Arg Asn Asp Val Met Val Ser Glu Trp Leu Asn Lys Leu
            340                 345                 350

Asn Leu Glu Glu Pro Pro Ser Ser Val Pro Lys Lys Cys Pro Ser Leu
        355                 360                 365

Thr Lys Arg Ser Arg Ala Gln Glu Glu Gln Val Pro Gln Ala Trp Thr
    370                 375                 380

Ala Gly Thr Ser Ser Asp Ser Met Ala Gln Pro Pro Gln Thr Pro Glu
385                 390                 395                 400

Thr Ser Thr Phe Arg Asn Gln Met Pro Ser Pro Thr Ser Thr Gly Thr
                405                 410                 415

Pro Ser Pro Gly Pro Arg Gly Asn Gln Gly Ala Glu Arg Gln Gly Met
            420                 425                 430

Asn Trp Ser Cys Arg Thr Pro Glu Pro Asn Pro Val Thr Gly Arg Pro
        435                 440                 445

Leu Val Asn Ile Tyr Asn Cys Ser Gly Val Gln Val Gly Asp Asn Asn
    450                 455                 460

Tyr Leu Thr Met Gln Gln Thr Thr Ala Leu Pro Thr Trp Gly Leu Ala
465                 470                 475                 480

Pro Ser Gly Lys Gly Arg Gly Leu Gln His Pro Pro Pro Val Gly Ser
                485                 490                 495

Gln Glu Gly Pro Lys Asp Pro Glu Ala Trp Ser Arg Pro Gln Gly Trp
            500                 505                 510

Tyr Asn His Ser Gly Lys
        515